# EUROPEAN PATENT APPLICATION

(11) **EP 4 785 954 A1**
(43) Date of publication of application: **05.08.2026**
(21) Application number: 24873042.6
(22) Date of filing: 27.09.2024
(51) Int. Cl.: A61K 39/395, A61K 9/08, A61K 47/12, A61K 47/26, A61K 47/18, A61K 47/02, A61K 9/00, A61P 19/02, A61P 29/00, A61P 17/06

(54) **STABLE LIQUID FORMULATION OF ANTI-IL-17 ANTIBODY**

(30) Priority: 27.09.2023 KR 20230130944
(71) Applicant: Samsung Bioepis Co., Ltd., Yeonsu-gu Incheon 21987 (KR)
(72) Inventor: NAM, Myung Joo, Incheon 21982 (KR); BOCK, Sung Je, Incheon 21982 (KR); KIM, In Ae, Seoul 05090 (KR); LEE, Hun Joo, Incheon 21996 (KR); CHANG, Sung Keun, Incheon 22003 (KR)
(74) Representative: Boult Wade Tennant LLP
(86) International application number: PCT/KR2024/014737
(87) International publication number: WO 2025/071335

(57) **Abstract**

The present disclosure relates to a liquid formulation comprising an anti-IL-17 antibody and a buffer, and having a pH of about 4.0 to about 7.0, a device comprising the same, and uses of the liquid formulation and device for treating IL-17-related conditions. The liquid formulation has excellent solubility and stability, and thus may be effectively used as a medicament for treating IL-17-related conditions.

## Description

### Technical Field

The present disclosure relates to a stable liquid formulation of an anti-IL-17 antibody, a device comprising the same, and a use thereof for treating IL-17-related conditions.

### Background Art

Unlike general protein drugs, antibody drugs have problems of physicochemical instability due to their larger molecular weights and more complex secondary/higher-order structures. Thus, there is a need to develop an optimal formulation that ensures quality and stability throughout the entire process of manufacture, storage, and administration to patients. Protein instability, caused by various external factors such as temperature, light, and chemical factors, may lead to a reduction in activity, thereby decreasing efficacy or inducing immunogenicity when administered to the human body. Therefore, it is important to reduce such instability of antibody drugs to maintain optimal quality thereof until administered to patients. Methods such as changing buffers, evaluating optimal pH, and adding stabilizers are used to achieve optimal quality. Because each antibody protein has unique physicochemical properties, optimal formulation conditions may vary, and it is necessary to derive a formulation suitable for a target substance.

Furthermore, in addition to ensuring the above-described stability, it is necessary to derive formulation components that, for patient convenience, minimize injection site pain(ISP) by removing or minimizing pain-inducing components (e.g., citric acid). In fact, Taltz^{®} (ixekizumab) was initially developed and approved as a citrate-containing formulation (pH 5.7, 20 mM citrate buffer, 200 mM NaCl, and 0.03 % PS80), and subsequently received approval for a citrate-free formulation (pH 5.2 to 6.2, 8 % sucrose, and 0.03 % PS80) in 2022.

Additionally, beyond protein aggregation, which is known as a key quality evaluation indicator for protein stability, liquid-liquid phase separation (LLPS) has been frequently observed in high-concentration antibody formulations. LLPS occurs when a homogenous protein solution separates into protein-rich and protein-poor phases. While protein aggregation is typically accelerated at temperature above recommended storage conditions, LLPS is reported to occur under refrigerated conditions. Thus, LLPS represents a stability risk during product storage prior to patient administration When uncontrolled, LLPS may lead to protein instability, comprising irreversible structural alterations and increased protein aggregation. Therefore, it is important to develop formulations in which LLPS is effectively controlled. Although such phenomena do not occur in all proteins or antibody therapeutics, given that ixekizumab-specific LLPS has been identified in the prior art and solubility improvements have been continuously pursued, LLPS should be evaluated and addressed alongside stability and patient convenience.

### Disclosure of Invention

### Technical Problem

Accordingly, provided is a stable liquid formulation of an anti-IL-17 antibody such as ixekizumab. Specifically, provided is a formulation that is stable throughout manufacturing, storage, and prior to administration to patients, while additionally minimizing liquid-liquid phase separation (LLPS) to control physicochemical changes and eliminating components that cause injection site pain(ISP), thereby providing formulations with improved patient convenience.

According to an aspect of the present disclosure, a liquid formulation comprising: an anti-IL-17 antibody; and a buffer, wherein a pH is about 4.0 to about 7.0.

According to another aspect of the present disclosure, a device comprising the liquid formulation.

According to another aspect of the present disclosure, a method of treating an IL-17-related condition, the method comprising administering the liquid formulation to a subject in need thereof.

According to another aspect of the present disclosure, a use of the liquid formulation in the manufacture of a medicament for treating an IL-17-related condition.

### Solution to Problem

Unless otherwise defined, all technical terms used herein are used with the same meaning commonly understood by one of ordinary skill in the art to which the present disclosure belongs. In addition, although preferred methods and materials are described in the specifications, those similar or equivalent thereto may also be regarded within the scope of the present disclosure. In addition, throughout the specification, all numerical values set forth herein are considered to comprise the meaning of "about" even if not explicitly stated. The contents of all publications cited as references herein are hereby incorporated by reference in their entirety.

In the present specification, the term "about" or "approximately" may generally be interpreted to comprise values or ranges within 10 %, 5 %, 4 %, 3 %, 2 %, or 1 % above and below a given value or range.

According to an aspect, a stable liquid formulation of an anti-IL-17 antibody, specifically, a liquid formulation comprising:
an anti-IL-17 antibody; and
a buffer,
wherein the pH is about 4.0 to about 7.0.

### (1) Antibody

As used herein, the term "antibody" may be interpreted to comprise a full-length antibody or an antigen-binding fragment thereof. The antibody comprises monoclonal antibodies, polyclonal antibodies, humanized antibodies, fully human antibodies, and chimeric antibodies.

The term "antigen-binding fragment" refers to a fragment comprising an antigen-binding region of an antibody. For example, the antigen-binding fragment comprises, but is not limited to, Fab fragments, F(ab')₂ fragments, Fc fragments, or scFv fragments.

In the present specification, the antibody may be an anti-IL-17 antibody. The anti-IL-17 antibody may refers to any antibody that specifically binds to interleukin-23 (IL-17). The IL-17 family comprises IL-17A, IL-17B, IL-17C, IL-17D, IL-17E, and IL-17F, and IL-17E is also known as IL-25.

The anti-IL-17 antibody may be an anti-IL-17A antibody.

The anti-IL-17 antibody may be ixekizumab (CAS No. 1143503-69-8) or an antibody having the same sequence. Therefore, the liquid formulation may be a stable liquid formulation of ixekizumab. Ixekizumab is a human immunoglobulin G4 (human IgG4) monoclonal antibody that binds to IL-17. Ixekizumab is marketed under the trade name Taltz^{®}. After being approved by the U.S. Food and Drug Administration (FDA) as a therapeutic agent for plaque psoriasis, and subsequently has been used for treatment of ankylosing spondylitis (AS), psoriatic arthritis, non-radiographic axial spondyloarthritis medication (nr-axSpA), and the like. The sequence of ixekizumab is known in the art and may be produced by conventional methods known in the art. More detailed information on ixekizumab may be readily obtained by those skilled in the art from publicly available databases.

As used herein, the term "ixekizumab" may also be interpreted to comprise ixekizumab in which the amino acid sequence is modified (deleted, inserted, and/or substituted), and/or whose glycosylation characteristics are modified, to the extent that polypeptide functions are not affected.

In addition, the anti-IL-17 antibody may be contained in the liquid formulation in a therapeutically effective amount.

A concentration of the anti-IL-17 antibody may be about 1 mg/ml to about 300 mg/ml, about 1 mg/ml to about 250 mg/ml, about 1 mg/ml to about 200 mg/ml, about 1 mg/ml to about 175 mg/ml, about 1 mg/ml to about 160 mg/ml, about 1 mg/ml to about 150 mg/ml, about 1 mg/ml to about 125 mg/ml, about 1 mg/ml to about 100 mg/ml, about 1 mg/ml to about 90 mg/ml, about 1 mg/ml to about 80 mg/ml, about 1 mg/ml to about 75 mg/ml, about 1 mg/ml to about 50 mg/ml, about 5 mg/ml to about 300 mg/ml, about 5 mg/ml to about 250 mg/ml, about 5 mg/ml to about 200 mg/ml, about 5 mg/ml to about 175 mg/ml, about 5 mg/ml to about 160 mg/ml, about 5 mg/ml to about 150 mg/ml, about 5 mg/ml to about 125 mg/ml, about 5 mg/ml to about 100 mg/ml, about 5 mg/ml to about 90 mg/ml, about 5 mg/ml to about 80 mg/ml, about 5 mg/ml to about 75 mg/ml, about 5 mg/ml to about 50 mg/ml, about 10 mg/ml to about 300 mg/ml, about 10 mg/ml to about 250 mg/ml, about 10 mg/ml to about 200 mg/ml, about 10 mg/ml to about 175 mg/ml, about 10 mg/ml to about 160 mg/ml, about 10 mg/ml to about 150 mg/ml, about 10 mg/ml to about 125 mg/ml, about 10 mg/ml to about 100 mg/ml, about 10 mg/ml to about 90 mg/ml, about 10 mg/ml to about 80 mg/ml, about 10 mg/ml to about 75 mg/ml, about 10 mg/ml to about 50 mg/ml, about 25 mg/ml to about 300 mg/ml, about 25 mg/ml to about 250 mg/ml, about 25 mg/ml to about 200 mg/ml, about 25 mg/ml to about 175 mg/ml, about 25 mg/ml to about 160 mg/ml, about 25 mg/ml to about 150 mg/ml, about 25 mg/ml to about 125 mg/ml, about 25 mg/ml to about 100 mg/ml, about 25 mg/ml to about 90 mg/ml, about 25 mg/ml to about 80 mg/ml, about 25 mg/ml to about 75 mg/ml, about 25 mg/ml to about 50 mg/ml, about 50 mg/ml to about 300 mg/ml, about 50 mg/ml to about 250 mg/ml, about 50 mg/ml to about 200 mg/ml, about 50 mg/ml to about 175 mg/ml, about 50 mg/ml to about 160 mg/ml, about 50 mg/ml to about 150 mg/ml, about 50 mg/ml to about 125 mg/ml, about 50 mg/ml to about 100 mg/ml, about 25 mg/ml to about 90 mg/ml, about 25 mg/ml to about 80 mg/ml, about 50 mg/ml to about 75 mg/ml, about 70 mg/ml to about 300 mg/ml, about 70 mg/ml to about 250 mg/ml, about 70 mg/ml to about 200 mg/ml, about 70 mg/ml to about 170 mg/ml, about 70 mg/ml to about 160 mg/ml, about 70 mg/ml to about 150 mg/ml, about 70 mg/ml to about 125 mg/ml, about 70 mg/ml to about 100 mg/ml, about 70 mg/ml to about 90 mg/ml, about 70 mg/ml to about 80 mg/ml, about 80 mg/ml to about 300 mg/ml, about 80 mg/ml to about 250 mg/ml, about 80 mg/ml to about 200 mg/ml, about 80 mg/ml to about 180 mg/ml, about 80 mg/ml to about 160 mg/ml, about 80 mg/ml to about 150 mg/ml, about 80 mg/ml to about 125 mg/ml, about 80 mg/ml to about 100 mg/ml, about 80 mg/ml to about 90 mg/ml, about 90 mg/ml to about 300 mg/ml, about 90 mg/ml to about 250 mg/ml, about 90 mg/ml to about 200 mg/ml, about 90 mg/ml to about 175 mg/ml, about 90 mg/ml to about 160 mg/ml, about 90 mg/ml to about 150 mg/ml, about 90 mg/ml to about 125 mg/ml, about 90 mg/ml to about 110 mg/ml, about 90 mg/ml to about 100 mg/ml, about 100 mg/ml to about 300 mg/ml, about 100 mg/ml to about 250 mg/ml, about 100 mg/ml to about 200 mg/ml, about 100 mg/ml to about 175 mg/ml, about 100 mg/ml to about 160 mg/ml, about 100 mg/ml to about 150 mg/ml, about 100 mg/ml to about 125 mg/ml, or about 100 mg/ml to about 110 mg/ml.

In one embodiment, the concentration of the anti-IL-17 antibody may be about 5 mg/ml, about 10 mg/ml, about 25 mg/ml, about 50 mg/ml, about 80 mg/ml, about 100 mg/ml, about 125 mg/ml, about 150 mg/ml, about 160 mg/ml, about 175 mg/ml, about 200 mg/ml, about 250 mg/ml, or about 300 mg/ml.

In certain embodiment, the concentration of the anti-IL-17 antibody may be about 25 mg/ml to about 200 mg/ml.

In certain embodiment, the concentration of the anti-IL-17 antibody may be about 50 mg/ml to about 150 mg/ml.

In certain embodiment, the concentration of the anti-IL-17 antibody may be about 80 mg/ml.

### (2) Buffer and pH

The liquid formulation according to an aspect comprises a buffer. The buffer may serve to adjust a pH of the liquid formulation. The pH of the liquid formulation may be maintained at a certain value or within a certain range by the buffer. Accordingly, the buffer may serve to provide the certain value or the certain range of the pH to the liquid formulation.

The buffer may be used regardless of type thereof as long as the buffer is applicable to a biopharmaceutical.

The buffer may comprise one or more selected from acetate, histidine, phosphate, citrate, succinate, malate, tartrate, carbonate, salts thereof, and hydrates thereof.

The term "salt" may be a pharmaceutically acceptable salt. The salt may comprise an inorganic acid salt, an organic acid salt, a metal salt, and the like, of a compound. The inorganic acid salt may be a hydrochloride, a bromate, a phosphate, a sulfate, or a bisulfate. The organic acid salt may be a formate, an acetate, a propionate, a lactate, an oxalate, a tartrate, a malate, a maleate, a citrate, a fumarate, a besylate, a camsylate, an edisylate, a trichloroacetate, a trifluoroacetate, a benzoate, a gluconate, a methanesulfonate, a glycolate, a succinate, a 4-toluenesulfonate, a galacturonate, an embonate, a glutamate, an ethanesulfonate, a benzenesulfonate, a p-toluenesulfonate, or an aspartate. The metal salt may be a calcium salt, a sodium salt, a magnesium salt, a strontium salt, or a potassium salt.

The term "hydrate" refers to a substance containing water molecules in the molecule. The hydrate may be a monohydrate, a dihydrate, or a trihydrate. For example, the hydrate of histidine may comprise histidine monohydrochloride monohydrate.

The pH of the liquid formulation according to an aspect may be about 4.0 to about 7.0. The pH of the liquid formulation may be provided by the buffer.

Specifically, the pH of the formulation may be any range or any value selected from about 4.0 to about 7.0. For example, the pH may be about 4.0 to about 7.0, about 4.0 to about 6.7, about 4.0 to about 6.5, about 4.0 to about 6.2, about 4.0 to about 6.0, about 4.0 to about 5.9, about 4.0 to about 5.8, about 4.0 to about 5.7, about 4.0 to about 5.5, about 4.5 to about 7.0, about 4.5 to about 6.7, about 4.5 to about 6.5, about 4.5 to about 6.2, about 4.5 to about 6.0, about 4.5 to about 5.9, about 4.5 to about 5.8, about 4.5 to about 5.7, about 4.5 to about 5.5, about 5.0 to about 7.0, about 5.0 to about 6.7, about 5.0 to about 6.5, about 5.0 to about 6.2, about 5.0 to about 6.0, about 5.0 to about 5.9, about 5.0 to about 5.8, about 5.0 to about 5.7, about 5.0 to about 5.5, about 5.1 to about 7.0, about 5.1 to about 6.7, about 5.1 to about 6.5, about 5.1 to about 6.2, about 5.1 to about 6.0, about 5.1 to about 5.9, about 5.1 to about 5.8, about 5.1 to about 5.7, about 5.1 to about 5.5, about 5.3 to about 7.0, about 5.3 to about 6.7, about 5.3 to about 6.5, about 5.3 to about 6.2, about 5.3 to about 6.0, about 5.3 to about 5.9, about 5.3 to about 5.8, about 5.3 to about 5.7, about 5.3 to about 5.5, about 5.4 to about 7.0, about 5.4 to about 6.7, about 5.4 to about 6.5, about 5.4 to about 6.2, about 5.4 to about 6.0, about 5.4 to about 5.9, about 5.4 to about 5.8, about 5.4 to about 5.7, about 5.4 to about 5.5, about 5.5 to about 7.0, about 5.5 to about 6.7, about 5.5 to about 6.5, about 5.5 to about 6.2, about 5.5 to about 6.0, about 5.5 to about 5.9, about 5.5 to about 5.8, about 5.5 to about 5.7, about 5.6 to about 7.0, about 5.6 to about 6.7, about 5.6 to about 6.5, about 5.6 to about 6.2, about 5.6 to about 6.0, about 5.6 to about 5.9, about 5.6 to about 5.8, about 5.6 to about 5.7, about 5.7 to about 7.0, about 5.7 to about 6.7, about 5.7 to about 6.5, about 5.7 to about 6.2, about 5.7 to about 6.0, about 5.7 to about 5.9, or about 5.7 to about 5.8.

In one embodiment, the pH of the liquid formulation may be about 3.5, about 4.0, about 4.5, about 5.0, about 5.5, about 5.8, about 5.9, about 6.0, about 6.1, about 6.2, about 6.3, about 6.4, about 6.5, about 6.6, about 6.7, about 6.8, about 6.9, about 7.0, about 7.1, about 7.2, about 7.3, about 7.4, about 7.5, about 7.6, about 7.7, about 7.8, about 7.9, about 8.0, about 8.1, about 8.2 about 8.3, about 8.4, or about 8.5.

In one embodiment, the pH of the liquid formulation may be any range or any value selected from the range of about 4.0 to about 6.5, about 4.0 to about 6.0, about 4.0 to about 5.8, about 4.0 to about 5.7, about 4.5 to about 6.5, about 4.5 to about 6.0, about 4.5 to about 5.8, about 4.5 to about 5.7, about 5.0 to about 6.5, about 5.0 to about 6.0, about 5.0 to about 5.8, about 5.0 to about 5.7, about 5.5 to about 6.5, about 5.5 to about 6.0, about 5.5 to about 5.8, about 5.5 to about 5.7, about 5.6 to about 6.5, about 5.6 to about 6.0, about 5.6 to about 5.8, about 5.6 to about 5.7, about 5.7 to about 6.5, about 5.7 to about 6.0, or about 5.7 to about 5.8.

In certain embodiment, the buffer may comprise one or more selected from acetate, histidine, citrate, succinate, salts thereof, and hydrates thereof.

In certain embodiment, the buffer may comprise one or more selected from acetate, histidine, succinate, salts thereof, and hydrates thereof.

In certain embodiment, the buffer may be free of citrate, a salt thereof and/or a hydrate thereof.

In certain embodiment, the buffer may: 1) be free of citrate, salts thereof, and/or hydrates thereof; and 2) comprise one or more selected from acetate, histidine, succinate, salts thereof, and hydrates thereof.

In certain embodiment, the buffer may: 1) be free of citrate, salts thereof and/or hydrates thereof; and 2) comprise one or more selected from acetate, succinate, salts thereof, and hydrates thereof.

In certain embodiment, the buffer may: 1)_be free of citrate, salts thereof and/or hydrates thereof; and 2) comprise one or more selected from acetate, salts thereof, and hydrates thereof.

In certain embodiment, the buffer may comprise one selected from acetate, salts thereof, and hydrates thereof. In certain embodiment, the buffer may comprise acetate, sodium acetate, or any combination thereof. In certain embodiment, the buffer may comprise sodium acetate. In the embodiments, the pH of the liquid formulation may be any range or any value selected from a range of about 3.5 to about 6.5, or about 4.0 to about 6.0, and for example, the pH may be 4.0, 4.5, 5.0, 5.5, or 6.0.

In certain embodiment, the buffer may comprise one or more selected from histidine, salts thereof, and hydrates thereof. In the embodiments, the pH of the liquid formulation may be any range or any value selected from a range of about 4.5 to about 6.5, or about 5.0 to about 6.0, and for example, the pH may be 5.0, 5.5, or 6.0.

In certain embodiment, the buffer may comprise one or more selected from citrate, salts thereof, and hydrates thereof. In certain embodiment, the buffer may comprise citrate, sodium citrate, or any combination thereof. In certain embodiment, the buffer may comprise sodium citrate. In the embodiments, the pH of the liquid formulation may be any range or any value selected from a range of about 4.0 to about 6.5, or about 4.5 to about 6.0, and for example, the pH may be 4.5, 5.0, 5.5, or 6.0.

In certain embodiment, the buffer may comprise one or more selected from succinate, salts thereof, and hydrates thereof. In certain embodiment, the buffer may comprise succinate, sodium succinate, or any combination thereof. In certain embodiment, the buffer may comprise sodium succinate. In the embodiments, the pH of the liquid formulation may be any range or any value selected from a range of about 4.0 to about 6.5, or about 4.5 to about 6.0, and for example, the pH may be 4.5, 5.0, 5.5, or 6.0.

In certain embodiment, specifically, opalescence and/or phase separation may not occur in the liquid formulation, and a combination of the buffer and the pH may be selected from the followings in which:
A) the buffer comprises acetate, and the pH is about 4.0 to about 6.0;
B) the buffer comprises histidine, and the pH is about 5.0 to about 6.0;
C) the buffer comprises citrate, and the pH is about 4.5 to about 6.0; or
D) the buffer comprises succinate, and the pH is about 4.5 to about 6.0.

In certain embodiment, specifically, the liquid formulation may have an aggregation temperature (T_{agg}) of about 66 °C or higher, and a combination of the buffer and the pH may be selected from the followings in which:
A) the buffer comprises acetate, and the pH is about 5.5 to about 6.0;
B) the buffer comprises histidine, and the pH is about 6.0;
C) the buffer comprises citrate, and the pH is about 6.0; or
D) the buffer comprises succinate, and the pH is about 6.0.

In certain embodiment, specifically, the liquid formulation may have an aggregation temperature (T_{agg}) of about 68 °C or higher, and a combination of the buffer and the pH may be selected from the followings in which:
A) the buffer comprises acetate, and the pH is about 6.0;
B) the buffer comprises citrate, and the pH is about 6.0; or
C) the buffer comprises succinate, and the pH is about 6.0.

The concentration of the buffer may be any range or any value selected from about 1 mM to about 60 mM. For example, the concentration of the buffer may be about 1 mM to about 60 mM, about 1 mM to about 50 mM, about 1 mM to about 40 mM, about 1 mM to about 35 mM, about 1 mM to about 34 mM, about 1 mM to about 33 mM, about 1 mM to about 32 mM, about 1 mM to about 31 mM, about 1 mM to about 30 mM, about 5 mM to about 60 mM, about 5 mM to about 50 mM, about 5 mM to about 40 mM, about 5 mM to about 35 mM, about 5 mM to about 34 mM, about 5 mM to about 33 mM, about 5 mM to about 32 mM, about 5 mM to about 31 mM, about 5 mM to about 30 mM, about 10 mM to about 60 mM, about 10 mM to about 50 mM, about 10 mM to about 40 mM, about 10 mM to about 35 mM, about 10 mM to about 34 mM, about 10 mM to about 33 mM, about 10 mM to about 32 mM, about 10 mM to about 31 mM, about 10 mM to about 30 mM, about 15 mM to about 60 mM, about 15 mM to about 50 mM, about 15 mM to about 40 mM, about 15 mM to about 35 mM, about 15 mM to about 34 mM, about 15 mM to about 33 mM, about 15 mM to about 32 mM, about 15 mM to about 31 mM, about 15 mM to about 30 mM, about 20 mM to about 60 mM, about 20 mM to about 50 mM, about 20 mM to about 40 mM, about 20 mM to about 35 mM, about 20 mM to about 34 mM, about 20 mM to about 33 mM, about 20 mM to about 32 mM, about 20 mM to about 31 mM, about 20 mM to about 30 mM, about 25 mM to about 60 mM, about 25 mM to about 50 mM, about 25 mM to about 40 mM, about 25 mM to about 35 mM, about 25 mM to about 34 mM, about 25 mM to about 33 mM, about 25 mM to about 32 mM, about 25 mM to about 31 mM, about 25 mM to about 30 mM, about 28 mM to about 60 mM, about 28 mM to about 50 mM, about 28 mM to about 40 mM, about 28 mM to about 35 mM, about 28 mM to about 34 mM, about 28 mM to about 33 mM, about 28 mM to about 32 mM, about 28 mM to about 31 mM, about 28 mM to about 30 mM, about 29 mM to about 60 mM, about 29 mM to about 50 mM, about 29 mM to about 40 mM, about 29 mM to about 35 mM, about 29 mM to about 34 mM, about 29 mM to about 33 mM, about 29 mM to about 32 mM, about 29 mM to about 31 mM, about 29 mM to about 30 mM, about 30 mM to about 60 mM, about 30 mM to about 50 mM, about 30 mM to about 40 mM, about 30 mM to about 35 mM, about 30 mM to about 34 mM, about 30 mM to about 33 mM, about 30 mM to about 32 mM, or about 30 mM to about 31 mM.

In certain embodiment, the concentration of the buffer may be about 20 mM, about 25 mM, about 28 mM, about 29 mM, about 30 mM, about 31 mM, about 32 mM, about 35 mM, or about 40 mM.

### (3) Stabilizer

A liquid formulation according to an aspect may further comprise a stabilizer. Accordingly, the liquid formulation may comprise: an anti-IL-17 antibody; a buffer; and a stabilizer and have a pH of about 4.0 to about 7.0.

The term "stabilizer" refers to a substance added to prevent a state change or a chemical change when leaving a substance to stand or preserving a substance.

The stabilizer may be used regardless of type thereof, as long as the stabilizer is applicable to biopharmaceuticals.

The stabilizer may comprise one or more selected from a sugar, a sugar alcohol, an amino acid, a metal salt, a salt thereof, and a hydrate thereof.

The sugar may be a monosaccharide, a disaccharide, an oligosaccharide, or a polysaccharide. The sugar may comprise one or more selected from trehalose, sucrose, galactose, mannose, maltose, lactose, fructose, and glucose.

The sugar alcohol is produced by reducing the aldehyde or ketone group of a sugar to an alcohol group and is a general term for polyols having two or more hydroxyl groups. The sugar alcohol may comprise one or more selected from mannitol, sorbitol, xylitol, arabitol, erythritol, lactitol, maltitol, and inositol. The sugar alcohol comprises anhydrides of the sugar alcohol. For example, trehalose may comprise not only trehalose but also trehalose dihydrate.

The amino acid may comprise one or more selected from glycine, alanine, valine, leucine, isoleucine, proline, phenylalanine, tyrosine, tryptophan, serine, threonine, cysteine, methionine, asparagine, glutamine, lysine, arginine, histidine, aspartic acid, and glutamic acid.

The metal salt may comprise one or more selected from NaCl, KCl, NaF, KBr, NaBr, Na₂SO₄, NaSCN, CaCl₂, MgCl₂, and K₂SO₄.

A concentration of the sugar may be freely adjusted within a range that maintains the stability of the antibody, and may vary individually depending on each specific sugar type. The concentration of the sugar may be any range or any value selected from about 0.1 % (w/v) to about 20.0 % (w/v). For example, the concentration of the sugar may be about 1.0 % (w/v) to about 20.0 % (w/v), about 1.0 % (w/v) to about 15.0 % (w/v), about 1.0 % (w/v) to about 12.0 % (w/v), about 1.0 % (w/v) to about 11.0 % (w/v), about 1.0 % (w/v) to about 10.5 % (w/v), about 1.0 % (w/v) to about 10.0 % (w/v), about 1.0 % (w/v) to about 9.5 % (w/v), about 1.0 % (w/v) to about 9.0 % (w/v), about 1.0 % (w/v) to about 8.5 % (w/v), about 1.0 % (w/v) to about 8.0 % (w/v), about 1.0 % (w/v) to about 7.5 % (w/v), about 1.0 % (w/v) to about 7.0 % (w/v), about 1.0 % (w/v) to about 6.5 % (w/v), about 1.0 % (w/v) to about 6.0 % (w/v), about 1.0 % (w/v) to about 5.0 % (w/v), about 1.0 % (w/v) to about 4.5 % (w/v), about 4.0 % (w/v) to about 20.0 % (w/v), about 4.0 % (w/v) to about 15.0 % (w/v), about 4.0 % (w/v) to about 12.0 % (w/v), about 4.0 % (w/v) to about 11.0 % (w/v), about 4.0 % (w/v) to about 10.5 % (w/v), about 4.0 % (w/v) to about 10.0 % (w/v), about 4.0 % (w/v) to about 9.5 % (w/v), about 4.0 % (w/v) to about 9.0 % (w/v), about 4.0 % (w/v) to about 8.5 % (w/v), about 4.0 % (w/v) to about 8.0 % (w/v), about 4.0 % (w/v) to about 7.5 % (w/v), about 4.0 % (w/v) to about 7.0 % (w/v), about 4.0 % (w/v) to about 6.5 % (w/v), about 4.0 % (w/v) to about 6.0 % (w/v), about 4.0 % (w/v) to about 5.0 % (w/v), about 4.0 % (w/v) to about 4.5 % (w/v), about 4.5 % (w/v) to about 20.0 % (w/v), about 4.5 % (w/v) to about 15.0 % (w/v), about 4.5 % (w/v) to about 12.0 % (w/v), about 4.5 % (w/v) to about 11.0 % (w/v), about 4.5 % (w/v) to about 10.5 % (w/v), about 4.5 % (w/v) to about 10.0 % (w/v), about 4.5 % (w/v) to about 9.5 % (w/v), about 4.5 % (w/v) to about 9.0 % (w/v), about 4.5 % (w/v) to about 8.5 % (w/v), about 4.5 % (w/v) to about 8.0 % (w/v), about 4.5 % (w/v) to about 7.5 % (w/v), about 4.5 % (w/v) to about 7.0 % (w/v), about 4.5 % (w/v) to about 6.5 % (w/v), about 4.5 % (w/v) to about 6.0 % (w/v), about 4.5 % (w/v) to about 5.0 % (w/v), about 5.5 % (w/v) to about 20.0 % (w/v), about 5.5 % (w/v) to about 15.0 % (w/v), about 5.5 % (w/v) to about 12.0 % (w/v), about 5.5 % (w/v) to about 11.0 % (w/v), about 5.5 % (w/v) to about 10.5 % (w/v), about 5.5 % (w/v) to about 10.0 % (w/v), about 5.5 % (w/v) to about 9.5 % (w/v), about 5.5 % (w/v) to about 9.0 % (w/v), about 5.5 % (w/v) to about 8.5 % (w/v), about 5.5 % (w/v) to about 8.0 % (w/v), about 5.5 % (w/v) to about 7.5 % (w/v), about 5.5 % (w/v) to about 7.0 % (w/v), about 5.5 % (w/v) to about 6.5 % (w/v), about 5.5 % (w/v) to about 6.0 % (w/v), about 7.0 % (w/v) to about 20.0 % (w/v), about 7.0 % (w/v) to about 15.0 % (w/v), about 7.0 % (w/v) to about 12.0 % (w/v), about 7.0 % (w/v) to about 11.0 % (w/v), about 7.0 % (w/v) to about 10.5 % (w/v), about 7.0 % (w/v) to about 10.0 % (w/v), about 7.0 % (w/v) to about 9.5 % (w/v), about 7.0 % (w/v) to about 9.0 % (w/v), about 7.0 % (w/v) to about 8.5 % (w/v), about 7.0 % (w/v) to about 8.0 % (w/v), about 7.0 % (w/v) to about 7.5 % (w/v), about 7.5 % (w/v) to about 20.0 % (w/v), about 7.5 % (w/v) to about 15.0 % (w/v), about 7.5 % (w/v) to about 12.0 % (w/v), about 7.5 % (w/v) to about 11.0 % (w/v), about 7.5 % (w/v) to about 10.5 % (w/v), about 7.5 % (w/v) to about 10.0 % (w/v), about 7.5 % (w/v) to about 9.5 % (w/v), about 7.5 % (w/v) to about 9.0 % (w/v), about 7.5 % (w/v) to about 8.5 % (w/v), about 7.5 % (w/v) to about 8.0 % (w/v), about 8.0 % (w/v) to about 20.0 % (w/v), about 8.0 % (w/v) to about 15.0 % (w/v), about 8.0 % (w/v) to about 12.0 % (w/v), about 8.0 % (w/v) to about 11.0 % (w/v), about 8.0 % (w/v) to about 10.5 % (w/v), about 8.0 % (w/v) to about 10.0 % (w/v), about 8.0 % (w/v) to about 9.5 % (w/v), about 8.0 % (w/v) to about 9.0 % (w/v), about 8.0 % (w/v) to about 8.5 % (w/v), about 8.5 % (w/v) to about 20.0 % (w/v), about 8.5 % (w/v) to about 15.0 % (w/v), about 8.5 % (w/v) to about 12.0 % (w/v), about 8.5 % (w/v) to about 11.0 % (w/v), about 8.5 % (w/v) to about 10.5 % (w/v), about 8.5 % (w/v) to about 10.0 % (w/v), about 8.5 % (w/v) to about 9.5 % (w/v), about 8.5 % (w/v) to about 9.0 % (w/v), about 9.0 % (w/v) to about 20.0 % (w/v), about 9.0 % (w/v) to about 15.0 % (w/v), about 9.0 % (w/v) to about 12.0 % (w/v), about 9.0 % (w/v) to about 11.0 % (w/v), about 9.0 % (w/v) to about 10.5 % (w/v), about 9.0 % (w/v) to about 10.0 % (w/v), about 9.0 % (w/v) to about 9.5 % (w/v), about 10.0 % (w/v) to about 20.0 % (w/v), about 10.0 % (w/v) to about 15.0 % (w/v), about 10.0 % (w/v) to about 12.0 % (w/v), about 10.0 % (w/v) to about 11.0 % (w/v), about 10.0 % (w/v) to about 10.5 % (w/v), about 10.5 % (w/v) to about 20.0 % (w/v), about 10.5 % (w/v) to about 15.0 % (w/v), about 10.5 % (w/v) to about 12.0 % (w/v), or about 10.5 % (w/v) to about 11.0 % (w/v).

A concentration of the sugar alcohol may be freely adjusted within a range that maintains the stability of the antibody, and may vary individually depending on each specific sugar alcohol type. The concentration of the sugar alcohol may be any range or any value selected from about 1.0 % (w/v) to about 20.0 % (w/v). For example, the concentration of the sugar alcohol may be about 1.0 % (w/v) to about 20.0 % (w/v), about 1.0 % (w/v) to about 15.0 % (w/v), about 1.0 % (w/v) to about 10.0 % (w/v), about 1.0 % (w/v) to about 8.0 % (w/v), about 1.0 % (w/v) to about 6.0 % (w/v), about 1.0 % (w/v) to about 5.0 % (w/v), about 1.0 % (w/v) to about 4.5 % (w/v), about 1.0 % (w/v) to about 4.0 % (w/v), about 2.0 % (w/v) to about 20.0 % (w/v), about 2.0 % (w/v) to about 15.0 % (w/v), about 2.0 % (w/v) to about 10.0 % (w/v), about 2.0 % (w/v) to about 8.0 % (w/v), about 2.0 % (w/v) to about 6.0 % (w/v), about 2.0 % (w/v) to about 5.0 % (w/v), about 2.0 % (w/v) to about 4.5 % (w/v), about 2.0 % (w/v) to about 4.0 % (w/v), about 3.0 % (w/v) to about 20.0 % (w/v), about 3.0 % (w/v) to about 15.0 % (w/v), about 3.0 % (w/v) to about 10.0 % (w/v), about 3.0 % (w/v) to about 8.0 % (w/v), about 3.0 % (w/v) to about 6.0 % (w/v), about 3.0 % (w/v) to about 5.0 % (w/v), about 3.0 % (w/v) to about 4.5 % (w/v), about 3.0 % (w/v) to about 4.0 % (w/v), about 3.5 % (w/v) to about 20.0 % (w/v), about 3.5 % (w/v) to about 15.0 % (w/v), about 3.5 % (w/v) to about 10.0 % (w/v), about 3.5 % (w/v) to about 8.0 % (w/v), about 3.5 % (w/v) to about 6.0 % (w/v), about 3.5 % (w/v) to about 5.0 % (w/v), about 3.5 % (w/v) to about 4.5 % (w/v), about 3.5 % (w/v) to about 4.0 % (w/v), about 4.0 % (w/v) to about 20.0 % (w/v), about 4.0 % (w/v) to about 15.0 % (w/v), about 4.0 % (w/v) to about 10.0 % (w/v), about 4.0 % (w/v) to about 8.0 % (w/v), about 4.0 % (w/v) to about 6.0 % (w/v), about 4.0 % (w/v) to about 5.0 % (w/v), or about 4.0 % (w/v) to about 4.5 % (w/v).

A concentration of the amino acid may be freely adjusted within a range that maintains the stability of the antibody, and may vary individually depending on each specific amino acid type. The concentration of the amino acid may be any range or any value selected from about 1 mM to about 300 mM. For example, the concentration of the amino acid may be about 1 mM to about 300 mM, about 1 mM to about 250 mM, about 1 mM to about 200 mM, about 1 mM to about 150 mM, about 1 mM to about 140 mM, about 1 mM to about 130 mM, about 1 mM to about 125 mM, about 1 mM to about 100 mM, about 1 mM to about 70 mM, about 1 mM to about 50 mM, about 1 mM to about 49 mM, about 1 mM to about 20 mM, about 1 mM to about 15 mM, about 1 mM to about 13 mM, about 1 mM to about 12.5 mM, about 5 mM to about 300 mM, about 5 mM to about 250 mM, about 5 mM to about 200 mM, about 5 mM to about 150 mM, about 5 mM to about 140 mM, about 5 mM to about 130 mM, about 5 mM to about 125 mM, about 5 mM to about 100 mM, about 5 mM to about 70 mM, about 5 mM to about 50 mM, about 5 mM to about 49 mM, about 5 mM to about 20 mM, about 5 mM to about 15 mM, about 5 mM to about 13 mM, about 5 mM to about 12.5 mM, about 10 mM to about 300 mM, about 10 mM to about 250 mM, about 10 mM to about 200 mM, about 10 mM to about 150 mM, about 10 mM to about 140 mM, about 10 mM to about 130 mM, about 10 mM to about 125 mM, about 10 mM to about 100 mM, about 10 mM to about 70 mM, about 10 mM to about 50 mM, about 10 mM to about 49 mM, about 10 mM to about 20 mM, about 10 mM to about 15 mM, about 10 mM to about 13 mM, about 10 mM to about 12.5 mM, about 12 mM to about 300 mM, about 12 mM to about 250 mM, about 12 mM to about 200 mM, about 12 mM to about 150 mM, about 12 mM to about 140 mM, about 12 mM to about 130 mM, about 12 mM to about 125 mM, about 12 mM to about 100 mM, about 12 mM to about 70 mM, about 12 mM to about 50 mM, about 12 mM to about 49 mM, about 12 mM to about 20 mM, about 12 mM to about 15 mM, about 12 mM to about 13 mM, or about 12 mM to about 12.5 mM.

A concentration of the metal salt may be freely adjusted within a range that maintains the stability of the antibody, and may vary individually depending on each specific metal salt type. The concentration of the metal salt may be any range or any value selected from about 1 to about 300 mM. For example, the concentration of the metal salt may be about 1 to about 300 mM, about 1 to about 250 mM, about 1 to about 200 mM, about 1 to about 150 mM, about 1 to about 140 mM, about 1 to about 137 mM, about 10 to about 300 mM, about 10 to about 250 mM, about 10 to about 200 mM, about 10 to about 150 mM, about 10 to about 140 mM, about 10 to about 137 mM, about 50 to about 300 mM, about 50 to about 250 mM, about 50 to about 200 mM, about 50 to about 150 mM, about 50 to about 140 mM, about 50 to about 137 mM, about 100 to about 300 mM, about 100 to about 250 mM, about 100 to about 200 mM, about 100 to about 150 mM, about 100 to about 140 mM, about 100 to about 137 mM, about 130 to about 300 mM, about 130 to about 250 mM, about 130 to about 200 mM, about 130 to about 150 mM, about 130 to about 140 mM, about 130 to about 137 mM, about 135 to about 300 mM, about 135 to about 250 mM, about 135 to about 200 mM, about 135 to about 150 mM, about 135 to about 140 mM, or about 135 to about 137 mM.

In one embodiment, the stabilizer may comprise one or more selected from trehalose, mannitol, arginine, lysine, histidine, methionine, glycine, and sodium chloride.

In one embodiment, the stabilizer may comprise one or more selected trehalose, mannitol, arginine, and lysine.

In one embodiment, the stabilizer may comprise one or more selected trehalose, arginine, and lysine.

In certain embodiment, the stabilizer may comprise trehalose. A concentration of the trehalose may be about 3.0 to about 12.0 %, about 3.0 to about 11.0 %, about 3.0 to about 10.5 %, about 3.0 to about 10.0 %, about 3.0 to about 9.5 %, about 3.0 to about 9.0 %, about 3.0 to about 8.0 %, about 3.0 to about 7.5 %, about 3.0 to about 7.0 %, about 3.0 to about 6.5 %, about 3.0 to about 6.0 %, about 3.0 to about 5.0 %, about 3.0 to about 4.5 %, about 4.5 to about 12.0 %, about 4.5 to about 11.0 %, about 4.5 to about 10.5 %, about 4.5 to about 10.0 %, about 4.5 to about 9.5 %, about 4.5 to about 9.0 %, about 4.5 to about 8.0 %, about 4.5 to about 7.5 %, about 4.5 to about 7.0 %, about 4.5 to about 6.5 %, about 4.5 to about 6.0 %, about 4.5 to about 5.0 %, about 5.0 to about 12.0 %, about 5.0 to about 11.0 %, about 5.0 to about 10.5 %, about 5.0 to about 10.0 %, about 5.0 to about 9.5 %, about 5.0 to about 9.0 %, about 5.0 to about 8.0 %, about 5.0 to about 7.5 %, about 5.0 to about 7.0 %, about 5.0 to about 6.5 %, about 5.0 to about 6.0 %, about 5.5 to about 12.0 %, about 5.5 to about 11.0 %, about 5.5 to about 10.5 %, about 5.5 to about 10.0 %, about 5.5 to about 9.5 %, about 5.5 to about 9.0 %, about 5.5 to about 8.0 %, about 5.5 to about 7.5 %, about 5.5 to about 7.0 %, about 5.5 to about 6.5 %, about 5.5 to about 6.0 %, about 6.0 to about 12.0 %, about 6.0 to about 11.0 %, about 6.0 to about 10.5 %, about 6.0 to about 10.0 %, about 6.0 to about 9.5 %, about 6.0 to about 9.0 %, about 6.0 to about 8.0 %, about 6.0 to about 7.5 %, about 6.0 to about 7.0 %, about 6.0 to about 6.5 %, about 7.0 to about 12.0 %, about 7.0 to about 11.0 %, about 7.0 to about 10.5 %, about 7.0 to about 10.0 %, about 7.0 to about 9.5 %, about 7.0 to about 9.0 %, about 7.0 to about 8.0 %, or about 7.0 to about 7.5 %.

In certain embodiment, the stabilizer may comprise mannitol. A concentration of the mannitol may be about 1.0 to about 10.0 % (w/v), about 1.0 to about 6.0 % (w/v), about 1.0 to about 4.5 % (w/v), about 1.0 to about 4.0 % (w/v), about 1.0 to about 3.0 % (w/v), about 2.0 to about 10.0 % (w/v), about 2.0 to about 6.0 % (w/v), about 2.0 to about 4.5 % (w/v), about 2.0 to about 4.0 % (w/v), about 2.0 to about 3.0 % (w/v), about 3.0 to about 10.0 % (w/v), about 3.0 to about 6.0 % (w/v), about 3.0 to about 4.5 % (w/v), about 3.0 to about 4.0 % (w/v), about 3.5 to about 10.0 % (w/v), about 3.5 to about 6.0 % (w/v), about 3.5 to about 4.5 % (w/v), about 3.5 to about 4.0 % (w/v), about 4.0 to about 10.0 % (w/v), about 4.0 to about 6.0 % (w/v), or about 4.0 to about 4.5 % (w/v).

In certain embodiment, the stabilizer may comprise lysine. A concentration of the lysine may be about 5 to about 20 mM, about 5 to about 18 mM, about 5 to about 17.5 mM, about 5 to about 15.5 mM, about 5 to about 15 mM, about 5 to about 13 mM, about 5 to about 12.5 mM, about 5 to about 10 mM, about 5 to about 8 mM, about 5 to about 7.5 mM, about 7 to about 20 mM, about 7 to about 18 mM, about 7 to about 17.5 mM, about 7 to about 15.5 mM, about 7 to about 15 mM, about 7 to about 13 mM, about 7 to about 12.5 mM, about 7 to about 10 mM, about 7 to about 8 mM, about 7 to about 7.5 mM, about 7.5 to about 20 mM, about 7.5 to about 18 mM, about 7.5 to about 17.5 mM, about 7.5 to about 15.5 mM, about 7.5 to about 15 mM, about 7.5 to about 13 mM, about 7.5 to about 12.5 mM, about 7.5 to about 10 mM, about 7.5 to about 8 mM, about 10 to about 20 mM, about 10 to about 18 mM, about 10 to about 17.5 mM, about 10 to about 15.5 mM, about 10 to about 15 mM, about 10 to about 13 mM, about 10 to about 12.5 mM, about 12 to about 20 mM, about 12 to about 18 mM, about 12 to about 17.5 mM, about 12 to about 15.5 mM, about 12 to about 15 mM, about 12 to about 13 mM, about 12 to about 12.5 mM, about 12.5 to about 20 mM, about 12.5 to about 18 mM, about 12.5 to about 17.5 mM, about 12.5 to about 15.5 mM, about 12.5 to about 15 mM, or about 12.5 to about 13 mM.

In certain embodiment, the stabilizer may comprise arginine. A concentration of the arginine may be about 50 to about 200 mM, about 50 to about 175 mM, about 50 to about 150 mM, about 50 to about 140 mM, about 50 to about 130 mM, about 50 to about 125 mM, about 75 to about 200 mM, about 75 to about 175 mM, about 75 to about 150 mM, about 75 to about 140 mM, about 75 to about 130 mM, about 75 to about 125 mM, about 100 to about 200 mM, about 100 to about 175 mM, about 100 to about 150 mM, about 100 to about 140 mM, about 100 to about 130 mM, about 100 to about 125 mM, about 110 to about 200 mM, about 110 to about 175 mM, about 110 to about 150 mM, about 110 to about 140 mM, about 110 to about 130 mM, about 110 to about 125 mM, about 120 to about 200 mM, about 120 to about 175 mM, about 120 to about 150 mM, about 120 to about 140 mM, about 120 to about 130 mM, about 120 to about 125 mM, about 125 to about 200 mM, about 125 to about 175 mM, about 125 to about 150 mM, about 125 to about 140 mM, or about 125 to about 130 mM.

In certain embodiment, the stabilizer may comprise histidine. A concentration of the histidine may be about 50 to about 90 mM, about 50 to about 80 mM, about 50 to about 75 mM, about 50 to about 70 mM, about 60 to about 90 mM, about 60 to about 80 mM, about 60 to about 75 mM, about 60 to about 70 mM, about 65 to about 90 mM, about 65 to about 80 mM, about 65 to about 75 mM, about 65 to about 70 mM, about 70 to about 90 mM, about 70 to about 80 mM, or about 70 to about 75 mM.

In certain embodiment, the stabilizer may comprise methionine. A concentration of the methionine may be about 70 to about 110 mM, about 70 to about 100 mM, about 70 to about 95 mM, about 70 to about 90 mM, about 80 to about 110 mM, about 80 to about 100 mM, about 80 to about 95 mM, about 80 to about 90 mM, about 85 to about 110 mM, about 85 to about 100 mM, about 85 to about 95 mM, about 85 to about 90 mM, about 90 to about 110 mM, about 90 to about 100 mM, or about 90 to about 95 mM.

In certain embodiment, the stabilizer may comprise glycine. A concentration of the glycine may be about 230 to about 270 mM, about 230 to about 260 mM, about 230 to about 255 mM, about 230 to about 250 mM, about 240 to about 270 mM, about 240 to about 260 mM, about 240 to about 255 mM, about 240 to about 250 mM, about 245 to about 270 mM, about 245 to about 260 mM, about 245 to about 255 mM, about 245 to about 250 mM, about 250 to about 270 mM, about 250 to about 260 mM, or about 250 to about 255 mM.

In certain embodiment, the stabilizer may comprise sodium chloride. A concentration of the sodium chloride may be about 100 to about 200 mM, about 100 to about 175 mM, about 100 to about 150 mM, about 100 to about 140 mM, about 100 to about 137 mM, about 125 to about 200 mM, about 125 to about 175 mM, about 125 to about 150 mM, about 125 to about 140 mM, about 125 to about 137 mM, about 130 to about 200 mM, about 130 to about 175 mM, about 130 to about 150 mM, about 130 to about 140 mM, about 130 to about 137 mM, about 135 to about 200 mM, about 135 to about 175 mM, about 135 to about 150 mM, about 135 to about 140 mM, about 135 to about 137 mM, about 137 to about 200 mM, about 137 to about 175 mM, about 137 to about 150 mM, or about 137 to about 140 mM.

A liquid formulation according to an aspect may be free of a stabilizer. As a result, the liquid formulation may comprise: an anti-IL-17 antibody; and a buffer, be free of a stabilizer, and have a pH of about 4.0 to about 7.0. The liquid formulation, even in a stabilizer-free state, may have superior stability to that of the stabilizer-containing Taltz^{®} formulation.

The term "free of component A" or "substantially free of component A" may be interpreted to comprise cases where the component A does not exist, or where, even though the component A exists, it is present in a trace amount that does not substantially affect characteristics of a formulation, or where the component A is present in an undetectable amount.

In the present specification, the phrase "free of stabilizer" may be interpreted as a case where a stabilizer component does not exist in a formulation, or is present in a negligible amount insufficient to provide any intended function as a stabilizer within the formulation.

In one embodiment, the stabilizer may be free of sucrose, trehalose, sorbitol, mannitol, arginine, lysine, histidine, methionine, glycine and/or sodium chloride.

In one embodiment, the stabilizer may be free of sucrose, trehalose, sorbitol, mannitol, lysine, methionine, glycine and/or sodium chloride.

In certain embodiment, the liquid formulation may be free of sucrose as a stabilizer.

In certain embodiment, the liquid formulation may be free of sodium chloride as a stabilizer.

### (4) Surfactant

The liquid formulation according to an aspect may further comprise a surfactant. Accordingly, the liquid formulation may comprise: an anti-IL-17 antibody; a buffer; and a surfactant, and have a pH of about 4.0 to about 7.0.

Accordingly, the liquid formulation may comprise: an anti-IL-17 antibody; a buffer; a stabilizer; and a surfactant, and have a pH of about 4.0 to about 7.0.

Accordingly, the liquid formulation may comprise: an anti-IL-17 antibody; a buffer; and a surfactant, be free of a stabilizer, and have a pH of about 4.0 to about 7.0.

The surfactant may be selected from any pharmaceutically acceptable surfactants capable of uniformly dispersing a protein (e.g.: antibody) in a liquid formulation medium.

The surfactant may be a non-ionic surfactant.

Specifically, the surfactant may be one or more selected from the group consisting of polysorbate, poloxamer, sorbitan esters of other fatty acids, polyethylene-polypropylene glycol, polyoxyethylene compounds, sodium dodecyl sulphate (SDS), and the like.

The polysorbate may comprise polysorbate 20, polysorbate 40, polysorbate 60, polysorbate 65, polysorbate 80, polysorbate 85, and the like.

The poloxamer may comprise a PEO-PPO-PEO copolymer, where PEO is poly(ethylene oxide) and PPO is poly(propylene oxide).

The sorbitan esters of other fatty acids may refer to sorbitan esters of fatty acids different from polysorbates, and may comprise, for example, sorbitan polyethoxylates.

The polyoxyethylene compound may comprise polyoxyethylene-stearate, polyoxyethylene alkyl ethers (where alkyl is C1-C30), polyoxyethylene monolauryl ether, alkylphenyl polyoxyethylene copolymers (where alkyl is C1-C30), and the like.

In one embodiment, the surfactant may comprise one or more selected from polysorbate 20, polysorbate 40, polysorbate 60, polysorbate 65, polysorbate 80, and polysorbate 85.

In one embodiment, the surfactant may comprise polysorbate 20, polysorbate 80, or any combination thereof.

In certain embodiment, the surfactant may comprise polysorbate 80.

A concentration of the surfactant may be any range or any value selected from about 0.001 % (w/v) to about 0.2 % (w/v). For example, the concentration of the surfactant may be about 0.001 % (w/v) to about 0.2 % (w/v), about 0.001 % (w/v) to about 0.1 % (w/v), about 0.001 % (w/v) to about 0.08 % (w/v), about 0.001 % (w/v) to about 0.06 % (w/v), about 0.001 % (w/v) to about 0.04 % (w/v), about 0.001 % (w/v) to about 0.03 % (w/v), about 0.005 % (w/v) to about 0.2 % (w/v), about 0.005 % (w/v) to about 0.1 % (w/v), about 0.005 % (w/v) to about 0.08 % (w/v), about 0.005 % (w/v) to about 0.06 % (w/v), about 0.005 % (w/v) to about 0.04 % (w/v), about 0.005 % (w/v) to about 0.03 % (w/v), about 0.01 % (w/v) to about 0.2 % (w/v), about 0.01 % (w/v) to about 0.1 % (w/v), about 0.01 % (w/v) to about 0.08 % (w/v), about 0.01 % (w/v) to about 0.06 % (w/v), about 0.01 % (w/v) to about 0.04 % (w/v), about 0.01 % (w/v) to about 0.03 % (w/v), about 0.02 % (w/v) to about 0.2 % (w/v), about 0.02 % (w/v) to about 0.1 % (w/v), about 0.02 % (w/v) to about 0.08 % (w/v), about 0.02 % (w/v) to about 0.06 % (w/v), about 0.02 % (w/v) to about 0.04 % (w/v), about 0.02 % (w/v) to about 0.03 % (w/v), about 0.03 % (w/v) to about 0.2 % (w/v), about 0.03 % (w/v) to about 0.1 % (w/v), about 0.03 % (w/v) to about 0.08 % (w/v), about 0.03 % (w/v) to about 0.06 % (w/v), or about 0.03 % (w/v) to about 0.04 % (w/v).

In certain embodiment, the concentration of the surfactant may be about 0.03 % (w/v).

### (5) Diluent

A liquid formulation according to an aspect may further comprise a diluent.

The diluent may be an aqueous carrier. The aqueous carrier is a pharmaceutically acceptable carrier that is safe and non-toxic when administered to humans, and may be, for example, water, a saline solution, Ringer's solution, dextrose, or any mixture thereof.

In one embodiment, the diluent may be water. The water may be water in a standard state. Therefore, the liquid formulation may be an aqueous liquid formulation.

The term "standard state" is a reference designating temperature and pressure used to describe a solution having certain composition, and may indicate, for example, a temperature of 25 °C ± 2 °C and a pressure of 1 atm. A person skilled in the art will recognize that a liquid formulation equivalent to the one disclosed in the present specification may be produced at other temperatures and pressures. Whether such a liquid formulation is equivalent to the one disclosed in the present specification may be determined under standard state conditions.

### (6) Solubility

The liquid formulation according to an aspect may be a Taltz^{®} biosimilar. Accordingly, the liquid formulation according to an aspect may have a similar or improved solubility compared to that of Taltz^{®}.

The term "solubility", as a solubility of an antibody contained in a formulation (e.g.: ixekizumab, refers to a level at which an antibody is dissolved in a liquid formulation immediately after manufacture, during storage, and before or after administration. The solubility may be confirmed by a state of the liquid formulation in which the antibody is dissolved, and specifically, may be confirmed by occurrence of opalescence, phase separation, or the like.

The liquid formulation may improve solubility of an antibody.

Liquid-liquid phase separation (LLPS) may not occur and/or may be minimized in the liquid formulation.

The term "liquid-liquid phase separation (LLPS)" refers to a phenomenon in which a high-concentration liquid protein is separated into a protein-rich bottom layer and a protein-poor top layer. While general protein aggregation is accelerated under increased temperature conditions compared to a storage temperature, the LLPS phenomenon is reported to occur under refrigerated conditions. The LLPS may occur in an environment where protein-protein interaction is strong, and may occur due to conditions such as a high-concentration formulation or a protein having a strong self-assembly tendency. The LLPS phenomenon may be one or more selected from opalescence and phase separation.

Opalescence and/or phase separation may not occur in the liquid formulation.

In certain embodiment, specifically, opalescence and/or phase separation may not occur in the liquid formulation, and a combination of the buffer and the pH may be selected from the followings in which:
A) the buffer comprises acetate, and the pH is about 4.0 to about 6.0;
B) the buffer comprises histidine, and the pH is about 5.0 to about 6.0;
C) the buffer comprises citrate, and the pH is about 4.5 to about 6.0; or
D) the buffer comprises succinate, and the pH is about 4.5 to about 6.0.

In one embodiment, opalescence and/or phase separation does not occur in the liquid formulation, and the liquid formulation may be selected from the followings in which:
A) the stabilizer comprises about 120 to about 160 mM of arginine;
B) the stabilizer comprises about 115 to about 155 mM of lysine;
C) the stabilizer comprises about 50 to about 90 mM of histidine;
D) the stabilizer comprises about 70 to about 110 mM of methionine;
E) the stabilizer comprises about 230 to about 270 mM of glycine; or
F) the formulation does not comprise a stabilizer.

### (7) Stability and Viscosity

The liquid formulation according to an aspect may be a Taltz^{®} biosimilar. Accordingly, the liquid formulation according to an aspect may have a stability and a viscosity improved compared to that of Taltz^{®}.

The liquid formulation according to an aspect may have a stability of an antibody while having a low viscosity.

In the embodiments of the present specification, two experiments were performed. First, thermal stability according to types of the buffer and the stabilizer was comparatively evaluated by measuring T_{agg}. Then, viscosity, and %HMW and a change therein (Δ%HMW - a change from week 0 to week 4) under various conditions were evaluated to comparatively evaluate a reduction in the viscosity and the stability according to a combination of the buffer and the stabilizer.

The term "biosimilar" is also referred to as a "biogeneric" and refers to a replica of original biopharmaceuticals. Because biopharmaceuticals are produced by cells rather than by synthesizing chemical products, it is impossible to replicate a product perfectly identical to the original medicament. Therefore, a replica of biopharmaceuticals is called a biosimilar in the sense that the replica is similar to, but not identical to, the original medicament.

The term "stability" indicates that an antibody (e.g.: ixekizumab) contained in a formulation substantially retains physical stability, chemical stability, and/or biological activity thereof before and after administration, during additional manufacturing processes, or during storage or preservation. Physical stability, chemical stability, and/or biological activity may be evaluated by commonly known methods. As a method for evaluating a stability of an antibody contained in a formulation in a short period of time, there is a method of measuring an aggregation temperature (T_{agg}) after exposing the formulation to heat (temperature) or a chemical substance, which are factors causing structural or physicochemical denaturation of a protein. For example, "improved stability" may indicate that a measured T_{agg} value is increased by 0.01 % or more, 0.1 % or more, 0.5 % or more, 1 % or more, 2 % or more, 3 % or more, 4 % or more, 5 % or more, 10 % or more, 15 % or more, or 20 % or more compared to that of a conventional formulation. An increase in the T_{agg} value may indicate that the stability is improved because a degree of aggregation or denaturation of the antibody in the formulation caused by external factors (e.g., heat, temperature, or chemical substances) is decreased.

The liquid formulation may inhibit aggregation of the antibody.

The liquid formulation may have improved stability and may have an increased aggregation temperature (T_{agg}).

The liquid formulation may have an increased T_{agg} compared to a liquid formulation which is free of a buffer and has a pH of 5.7. The liquid formulation may have an increased T_{agg} compared to a liquid formulation which is free of a buffer, comprises sucrose as a stabilizer, and has a pH of 5.7. The liquid formulation may have an increased T_{agg} compared to a liquid formulation which is free of a buffer, comprises 8 % sucrose as a stabilizer, and has a pH of 5.7.

The liquid formulation may have an aggregation temperature (T_{agg}) of higher than 65.86 °C, 65.87 °C or higher, 66 °C or higher, or 68 °C or higher.

In one embodiment, the liquid formulation may have an aggregation temperature (T_{agg}) of about 65.87 °C or higher or about 66 °C or higher, and may be selected from the followings in which:
A) the buffer comprises acetate, and the pH is about 5.5 to about 6.0;
B) the buffer comprises histidine, and the pH is about 6.0;
C) the buffer comprises citrate, and the pH is about 6.0; or
D) the buffer comprises succinate, and the pH is about 6.0.

In one embodiment, the liquid formulation may have an aggregation temperature (T_{agg}) of about 68 °C or higher, and may be selected from the followings in which:
A) the buffer comprises acetate, and the pH is about 6.0;
B) the buffer comprises citrate, and the pH is about 6.0; or
C) the buffer comprises succinate, and the pH is about 6.0.

In one embodiment, the liquid formulation may have an aggregation temperature (T_{agg}) of about 65.87 °C or higher or about 66 °C or higher, and may be selected from the followings in which:
A) the stabilizer comprises about 120 to about 160 mM of arginine;
B) the stabilizer comprises about 115 to about 155 mM of lysine;
C) the stabilizer comprises about 50 to about 90 mM of histidine;
D) the stabilizer comprises about 70 to about 110 mM of methionine;
E) the stabilizer comprises about 230 to about 270 mM of glycine; or
F) the formulation does not comprise a stabilizer.

In one embodiment, the liquid formulation may have an aggregation temperature (T_{agg}) of about 68 °C or higher, and may be selected from the followings in which:
A) the stabilizer comprises about 120 to about 160 mM of arginine;
B) the stabilizer comprises about 115 to about 155 mM of lysine;
C) the stabilizer comprises about 70 to about 110 mM of methionine;
D) the stabilizer comprises about 230 to about 270 mM of glycine; and
E) the formulation does not comprise a stabilizer.

The liquid formulation may have a T_{agg} increased by 0.1 % or more, 0.5 % or more, 0.8 % or more, 1 % or more, 3 % or more, 5 % or more, or 10 % or more compared to a liquid formulation which is free of a buffer and has a pH of 5.7, specifically a liquid formulation which is free of a buffer, comprises sucrose as a stabilizer, and has a pH of 5.7, and more specifically a liquid formulation which is free of a buffer, comprises 8 % sucrose as a stabilizer, and has a pH of 5.7.

The term "aggregate" may indicate a high molecular weight (HMW) species formed by aggregation of an antibody protein. The term "protein aggregation rate" may be expressed as a content ratio of the high molecular weight species (%HMW) of the antibody in the formulation at a certain point of time. The %HMW may be measured by size exclusion chromatography (SEC), but is not limited thereto. For example, "improved stability" may indicate that the measured %HMW of the antibody is decreased by 0.01 % or more, 0.1 % or more, 0.5 % or more, 1 % or more, 2 % or more, 3 % or more, 4 % or more, 5 % or more, 10 % or more, 15 % or more, or 20 % or more compared to that a conventional formulation. A decrease in the %HMW may indicate that the stability is improved because a degree of aggregation of the antibody in the formulation is decreased.

The stability evaluation may be performed immediately after manufacture of the formulation; or after storage for a certain period of time under accelerated stability conditions or stress stability conditions. The accelerated stability conditions may comprise conditions used in an accelerated test for a medicament, and may be, for example, a temperature of 25±2°C and a relative humidity (RH) of 60±5 %. The stress stability conditions may comprise conditions used in a stress test for a medicinal product, and may be, for example, a temperature of 40±2°C and a relative humidity (RH) of 75±5 %.

The liquid formulation has the improved stability and may have a decreased %HMW or Δ%HMW (a change from week 0 to week 4).

The liquid formulation may have a decreased %HMW or Δ%HMW compared to a liquid formulation which is free of a buffer and has a pH of 5.7. The liquid formulation may have a decreased %HMW or Δ%HMW compared to a liquid formulation which is free of a buffer, comprises sucrose as a stabilizer, and has a pH of 5.7. The liquid formulation may have a decreased %HMW or Δ%HMW compared to a liquid formulation which is free of a buffer, comprises 8 % sucrose as a stabilizer, and has a pH of 5.7. The liquid formulation may have a decreased %HMW or Δ%HMW under 25 °C and/or 40 °C conditions compared to a liquid formulation which is free of a buffer, comprises 8 % sucrose as a stabilizer, and has a pH of 5.7. The liquid formulation may have a decreased %HMW or Δ%HMW after storage under 25 °C and/or 40 °C conditions for 4 weeks compared to a liquid formulation which is free of a buffer, comprises 8 % sucrose as a stabilizer, and has a pH of 5.7.

The liquid formulation may have a %HMW of the antibody, measured after storage under 40 °C conditions for 4 weeks, of less than 4.3, 4.0 or less, 3.5 or less, 3.3 or less, specifically, 3.0 to less than 4.3, 3.0 to 4.0, 3.0 to 3.5, 3.0 to 3.3, 3.2 to less than 4.3, 3. 2 to 4.0, 3. 2 to 3.5, or 3.2 to 3.3.

The liquid formulation may have a %HMW of the antibody, measured after storage under 40 °C conditions for 4 weeks, of less than 4.3, 4.0 or less, or 3.5 or less, and may be selected from the followings in which:
A) the buffer comprises acetate, and the stabilizer comprises lysine and mannitol;
B) the buffer comprises 10 to 30 mM of acetate, the stabilizer comprises 40 to 60 mM of lysine and 2 to 4 % of mannitol, and the pH is about 5.5 to 6.5;
C) the buffer comprises acetate, and the stabilizer comprises lysine and trehalose;
D) the buffer comprises 10 to 30 mM of acetate, the stabilizer comprises 40 to 60 mM of lysine and 4 to 8 % of trehalose, and the pH is about 5.5 to 6.5;
E) the buffer comprises succinate, and the stabilizer comprises lysine and mannitol;
F) the buffer comprises 10 to 30 mM of succinate, the stabilizer comprises 40 to 60 mM of lysine and 2 to 4 % of mannitol, and the pH is about 5.5 to 6.5.

The liquid formulation may have a %HMW of an antibody, measured after storage under 25 °C for 4 weeks, or less than 2.3, 2.2 or less, or 2.1 or less, specifically 1.5 to less than 2.3, 1.5 to 2.2, 1.5 to 2.1, 1.8 to less than 2.3, 1.8 to 2.2, 1.8 to 2.1, 2.0 to less than 2.3, 2.0 to 2.2, or 2.0 to 2.1.

The liquid formulation may have a %HMW of the antibody, measured after storage under 25 °C conditions for 4 weeks, of less than 2.3, 2.2 or less, or 2.1 or less, and may be selected from the followings in which:
A) the buffer comprises acetate, and the stabilizer comprises lysine and mannitol;
B) the buffer comprises 10 to 30 mM of acetate, the stabilizer comprises 40 to 60 mM of lysine and 2 to 4 % of mannitol, and the pH is about 5.5 to 6.5;
C) the buffer comprises acetate, and the stabilizer comprises lysine and trehalose;
D) the buffer comprises 10 to 30 mM of acetate, the stabilizer comprises 40 to 60 mM of lysine and 4 to 8 % of trehalose, and the pH is about 5.5 to 6.5;
E) the buffer comprises succinate, and the stabilizer comprises lysine and mannitol; or
F) the buffer comprises 10 to 30 mM of succinate, the stabilizer comprises 40 to 60 mM of lysine and 2 to 4 % of mannitol, and the pH is about 5.5 to 6.5.

The liquid formulation may have a %HMW value of an antibody, measured after storage under 40 °C conditions for 4 weeks, decreased by 1 % or more, 5 % or more, 10 % or more, 15 % or more, or 20 % or more compared to a liquid formulation which is free of a buffer and has a pH of 5.7, specifically a liquid formulation which is free of a buffer, comprises sucrose as a stabilizer, and has a pH of 5.7, and more specifically a liquid formulation which is free of a buffer, comprises 8 % sucrose as a stabilizer, and has a pH of 5.7. For example, the value is decreased by 10 % to 40 %, 10 % to 35 %, 10 % to 30 %, 10 % to 25 %, 15 % to 40 %, 15 % to 35 %, 15 % to 30 %, 15 % to 25 %, 20 % to 40 %, 20 % to 35 %, 20 % to 30 %, or 20 % to 25 %.

The liquid formulation may have a %HMW value of an antibody, measured after storage under 25 °C conditions for 4 weeks, decreased by 1 % or more, 3 % or more, 5 % or more, or 8 or more compared to a liquid formulation which is free of a buffer and has a pH of 5.7, specifically a liquid formulation which is free of a buffer, comprises sucrose as a stabilizer, and has a pH of 5.7, and more specifically a liquid formulation which is free of a buffer, comprises 8 % sucrose as a stabilizer, and has a pH of 5.7. For example, the value is decreased by 1 % to 15 %, 1 % to 10 %, 5 % to 15 %, 5 % to 10 %, 8 % to 15 %, or 8 % to 10 %.

The liquid formulation may have a Δ%HMW of an antibody of less than 3.2, 3.0 or less, 2.8 or less, specifically 1.5 to less than 3.2, 1.5 to 3.0, 1.5 to 2.8, 1.5 to 2.5, 1.5 to 2.2, 1.5 to 2.0, 1.8 to less than 3.2, 1.8 to 3.0, 1.8 to 2.8, 1.8 to 2.5, 1.8 to 2.2, 1.8 to 2.0, 2.0 to less than 3.2, 2.0 to 3.0, 2.0 to 2.8, 2.0 to 2.5, or 2.0 to 2.2, when measured after storage under 40 °C conditions for 4 weeks.

The liquid formulation may have a Δ%HMW of less than 3.2, measured after storage under 40 °C conditions for 4 weeks, and may be selected from the followings in which:
A) the buffer comprises acetate, and the stabilizer comprises lysine and trehalose;
B) the buffer comprises 10 to 50 mM of acetate, the stabilizer comprises 5 to 20 mM of lysine and 3 to 12 % (w/v) of trehalose, and the pH is about 5.0 to 6.5;
C) the buffer comprises 20 to 50 mM of acetate, the stabilizer comprises 10 to 20 mM of lysine and 6 to 12 % (w/v) of trehalose, and the pH is about 5.5 to 6.0;
D) the buffer comprises acetate, and the stabilizer comprises lysine and mannitol;
E) the buffer comprises 10 to 50 mM of acetate, the stabilizer comprises 5 to 20 mM of lysine and 3.0 to 5.0 % (w/v) of mannitol, and the pH is about 5.0 to 6.5;
F) the buffer comprises 20 to 40 mM of acetate, the stabilizer comprises 10 to 15 mM of lysine and 3.0 to 5.0 % (w/v) of mannitol, and the pH is about 5.5 to 6.0;
G) the buffer comprises acetate, and the stabilizer comprises lysine and arginine;
H) the buffer comprises 10 to 50 mM of acetate, the stabilizer comprises 5 to 20 mM of lysine and 100 to 150 mM of arginine, and the pH is about 5.0 to 6.5; or

I) the buffer comprises 20 to 40 mM of acetate, the stabilizer comprises 10 to 15 mM of lysine and 120 to 140 mM of arginine, and the pH is about 5.5 to 6.0.

The liquid formulation may have a Δ%HMW of an antibody of 2.3 or less, when measured after storage under 40 °C conditions for 4 weeks, and may be selected from the followings in which:
A) the buffer comprises acetate, and the stabilizer comprises lysine and arginine;
B) the buffer comprises 20 to 40 mM of acetate, the stabilizer comprises 10 to 15 mM of lysine and 120 to 150 mM of arginine, and the pH is about 5.5 to 6.0; or
C) the buffer comprises 20 to 40 mM of acetate, the stabilizer comprises 10 to 15 mM of lysine and 120 to 140 mM of arginine, and the pH is about 5.5 to 6.0.

The liquid formulation may have a Δ%HMW of less than 1.2 1.0 or less, or 0.8 or less, specifically, 0.3 to less than 1.2, 0.3 to 1.0, 0.3 to 0.8, 0.3 to 0.5, 0.5 to less than 1.2, 0.5 to 1.0, or 0.5 to 0.8 when measured after storage under 25 °C conditions for 4 weeks,.

The liquid formulation may have a Δ%HMW of less than 1.2, when measured after storage under 25 °C conditions for 4 weeks, and may be selected from the followings in which:
A) the buffer comprises acetate, and the stabilizer comprises lysine and trehalose;
B) the buffer comprises 10 to 50 mM of acetate, the stabilizer comprises 5 to 20 mM of lysine and 3 to 12 % (w/v) of trehalose, and the pH is about 5.0 to 6.5;
C) the buffer comprises 20 to 50 mM of acetate, the stabilizer comprises 10 to 20 mM of lysine and 6 to 12 % (w/v) of trehalose, and the pH is about 5.5 to 6.0;
D) the buffer comprises acetate, and the stabilizer comprises lysine and mannitol;
E) the buffer comprises 10 to 50 mM of acetate, the stabilizer comprises 5 to 20 mM of lysine and 3.0 to 5.0 % (w/v) of mannitol, and the pH is about 5.0 to 6.5;
F) the buffer comprises 20 to 40 mM of acetate, the stabilizer comprises 10 to 15 mM of lysine and 3.0 to 5.0 % (w/v) of mannitol, and the pH is about 5.5 to 6.0;
G) the buffer comprises acetate, and the stabilizer comprises lysine and arginine;
H) the buffer comprises 10 to 50 mM of acetate, the stabilizer comprises 5 to 20 mM of lysine and 100 to 150 mM of arginine, and the pH is about 5.0 to 6.5; or

I) the buffer comprises 20 to 40 mM of acetate, the stabilizer comprises 10 to 15 mM of lysine and 120 to 140 mM of arginine, and the pH is about 5.5 to 6.0.

The liquid formulation may have a Δ%HMW of 0.5 or less, when measured after storage under 25 °C conditions for 4 weeks, and may be selected from the followings in which:
A) the buffer comprises acetate, and the stabilizer comprises lysine and arginine;
B) the buffer comprises 20 to 40 mM of acetate, the stabilizer comprises 10 to 15 mM of lysine and 100 to 150 mM of arginine, and the pH is about 5.5 to 6.0; or
C) the buffer comprises 20 to 40 mM of acetate, the stabilizer comprises 10 to 15 mM of lysine and 120 to 140 mM of arginine, and the pH is about 5.5 to 6.0.

The liquid formulation may have a Δ%HMW value of an antibody (%HMW of 4th week - %HMW of 0th week) decreased by 5 % or more, 10 % or more, 20 % or more, 30 % or more, or 35 % or more, when measured after storage under 40 °C conditions for 4 weeks, compared to a liquid formulation which is free of a buffer and has a pH of 5.7, specifically a liquid formulation which is free of a buffer, comprises sucrose as a stabilizer, and has a pH of 5.7, and more specifically a liquid formulation which is free of a buffer, comprises 8 % sucrose as a stabilizer, and has a pH of 5.7. For example, the Δ%HMW value may be decreased by 5 % to 45 %, 5 % to 40 %, 5 % to 30 %, 5 % to 20 %, 5 % to 15 %, 10 % to 45 %, 10 % to 40 %, 10 % to 30 %, 10 % to 20 %, 10 % to 15 %, 20 % to 45 %, 20 % to 40 %, 20 % to 30 %, 30 % to 45 %, 30 % to 40 %, 35 % to 45 %, or 35 % to 40 %.

The liquid formulation may have a Δ%HMW value of an antibody (%HMW of 4th week - %HMW of 0th week) decreased by 5 % or more, 10 % or more, 20 % or more, 25 % or more, 30 % or more, 40 % or more, 50 % or more, or 55 % or more, when measured after storage under 25 °C conditions for 4 weeks, compared to a liquid formulation which is free of a buffer and has a pH of 5.7, specifically, a liquid formulation which is free of a buffer, comprises sucrose as a stabilizer, and has a pH of 5.7, and more specifically, a liquid formulation which is free of a buffer, comprises 8% sucrose as a stabilizer, and has a pH of 5.7. For example, the Δ%HMW value may be decreased by 5 % to 60 %, 5 % to 50 %, 5 % to 40 %, 5 % to 30 %, 5 % to 25 %, 10 % to 60 %, 10 % to 50 %, 10 % to 40 %, 10 % to 30 %, 10 % to 25 %, 20 % to 60 %, 20 % to 50 %, 20 % to 40 %, 20 % to 30 %, 20 % to 25 %, 25 % to 60 %, 25 % to 50 %, 25 % to 40 %, 25 % to 30 %, 30 % to 60 %, 30 % to 50 %, 30 % to 40 %, 40 % to 60 %, 40 % to 50 %, or 50 % to 60 %.

The liquid formulation may have an improved viscosity. The 'improvement' of the viscosity may refer to 'reduction' in the viscosity. Therefore, the liquid formulation may have a reduced viscosity.

The liquid formulation may have a reduced viscosity compared to a liquid formulation which is free of a buffer and has a pH of 5.7. The liquid formulation may have a reduced viscosity compared to a liquid formulation which is free of a buffer, comprises sucrose as a stabilizer, and has a pH of 5.7. The liquid formulation may have a reduced viscosity compared to a liquid formulation which is free of a buffer, comprises 8 % sucrose as a stabilizer, and has a pH of 5.7.

The viscosity of the liquid formulation may be less than 6.0 centiPoise (cP). The viscosity of the liquid formulation may be less than 6.0 cP, 5.0 cP or less, 4.0 cP or less, 3.5 cP or less, or 3.0 cP or less. Specifically, the viscosity of the liquid formulation may be 2.0 cP to less than 6.0 cP, 2.0 cP to 5.0 cP, 2.0 cP to 4.0 cP, 2.0 cP to 3.5 cP, 2.0 cP to 3.0 cP, 2.5 cP to less than 6.0 cP, 2.5 cP to 5.0 cP, 2.5 cP to 4.0 cP, 2.5 cP to 3.5 cP, 2.5 cP to 3.0 cP, 3.0 cP to less than 6.0 cP, 3.0 cP to 5.0 cP, 3.0 cP to 4.0 cP, or 3.0 cP to 3.5 cP.

The liquid formulation may have a viscosity reduced by 10 % or more, 20 % or more, 30 % or more, 40 % or more, or 50 % or more, compared to a liquid formulation which is free of a buffer and has a pH of 5.7, specifically, a liquid formulation which is free of a buffer, comprises sucrose as a stabilizer, and has a pH of 5.7, and more specifically, a liquid formulation which is free of a buffer, comprises 8 % sucrose as a stabilizer, and has a pH of 5.7. For example, the liquid formulation may have a viscosity reduced by 10 % to 50 %, 10 % to 40 %, 10 % to 30 %, 10 % to 20 %, 20 % to 50 %, 20 % to 40 %, 20 % to 30 %, 30 % to 50 %, 30 % to 40 % or 40 % to 50 %.

### (8) Formulation

The term "liquid formulation" refers to a formulation in a liquid state.

A liquid formulation according to an aspect is a stable liquid formulation of an anti-IL-17 antibody. The liquid formulation may have improved solubility and/or stability compared to Taltz^{®}.

A liquid formulation according to an aspect may be selected from the items below:
1) a liquid formulation comprising: about 5 mg/mL to about 300 mg/mL of an anti-IL-17 antibody; about 10 mM to about 50 mM of acetate; about 7.5 mM to about 12.5 mM of lysine; and about 100 mM to about 150 mM of arginine, wherein a pH is about 5.4 to about 6.0;
2) a liquid formulation comprising: about 5 mg/mL to about 300 mg/mL of ixekizumab; about 10 mM to about 50 mM of acetate; about 7.5 mM to about 12.5 mM of lysine; and about 100 mM to about 150 mM of arginine, wherein a pH is about 5.4 to about 6.0;
3) a liquid formulation comprising: about 60 mg/mL to about 100 mg/mL of ixekizumab; about 20 mM to about 40 mM of acetate; about 10 mM to about 15 mM of lysine; and about 110 mM to about 140 mM of arginine, wherein a pH is about 5.6 to about 5.8;
4) a liquid formulation comprising: about 60 mg/mL to about 100 mg/mL of ixekizumab; about 20 mM to about 40 mM of acetate; about 10 mM to about 15 mM of lysine; about 110 mM to about 140 mM of arginine; and about 0.02 % (w/v) to about 0.04 % (w/v) of polysorbate 80, wherein a pH is about 5.6 to about 5.8;
5) a liquid formulation comprising about 80±5 mg/mL of ixekizumab; about 20±5 mM of acetate; about 12.5±1 mM of lysine; about 125±5 mM of arginine; about 0.03±0.005 % (w/v) of polysorbate 80, wherein a pH is about 5.7±0.05;
6) a liquid formulation comprising: about 5 mg/mL to about 300 mg/mL of an anti-IL-17 antibody; about 10 mM to about 50 mM of acetate; about 5 mM to about 20 mM of lysine; and about 3.0 % (w/v) to about 15.0 % (w/v) of trehalose, wherein a pH is about 5.4 to about 6.0;
7) a liquid formulation comprising: about 5 mg/mL to about 300 mg/mL of ixekizumab; about 10 mM to about 50 mM of acetate; about 5 mM to about 20 mM of lysine; and about 3.0 % (w/v) to about 15.0 % (w/v) of trehalose, wherein a pH is about 5.4 to about 6.0;
8) a liquid formulation comprising: about 60 mg/mL to about 100 mg/mL of ixekizumab; about 20 mM to about 50 mM of acetate; about 10 mM to about 20 mM of lysine; and about 5.0 % (w/v) to about 12.0 % (w/v) of trehalose, wherein a pH is about 5.5 to about 6.2;
9) a liquid formulation comprising: about 60 mg/mL to about 100 mg/mL of ixekizumab; about 20 mM to about 50 mM of acetate; about 10 mM to about 20 mM of lysine; about 5.0 % (w/v) to about 12.0 % (w/v) of trehalose; and about 0.02 % (w/v) to about 0.04 (w/v) of polysorbate 80, wherein a pH is about 5.5 to about 6.2;
10) a liquid formulation comprising: about 60 mg/mL to about 100 mg/mL of ixekizumab; about 20 mM to about 40 mM of acetate; about 10 mM to about 15 mM of lysine; about 5.0 % (w/v) to about 10.0 % (w/v) of trehalose; and about 0.02 % (w/v) to about 0.04 (w/v) of polysorbate 80, wherein a pH is about 5.6 to about 5.8;
11) a liquid formulation comprising about 80±5 mg/mL of ixekizumab; about 40±5 mM of acetate; about 15±1 mM of lysine; about 6.0±1 % (w/v) of trehalose; about 0.03±0.005 % (w/v) of polysorbate 80, wherein a pH is about 6.0±0.1;
12) a liquid formulation comprising about 80±5 mg/mL of ixekizumab; about 40±5 mM of acetate; about 10±1 mM of lysine; about 9.0±1 % (w/v) of trehalose; about 0.03±0.005 % (w/v) of polysorbate 80, wherein a pH is about 6.0±0.1;
13) a liquid formulation comprising about 80±5 mg/mL of ixekizumab; about 20±5 mM of acetate; about 15±1 mM of lysine; about 9.0±1 % (w/v) of trehalose; about 0.03±0.005 % (w/v) of polysorbate 80, wherein a pH is about 6.0±0.1;
14) a liquid formulation comprising about 80±5 mg/mL of ixekizumab; about 40±5 mM of acetate; about 15±1 mM of lysine; about 9.0±1 % (w/v) of trehalose; about 0.03±0.005 % (w/v) of polysorbate 80, wherein a pH is about 6.0±0.1;
15) a liquid formulation comprising about 80±5 mg/mL of ixekizumab; about 30±5 mM of acetate; about 12.5±1 mM of lysine; about 7.5±1 % (w/v) of trehalose; about 0.03±0.005 % (w/v) of polysorbate 80, wherein a pH is about 5.7±0.1;
16) a liquid formulation comprising about 80±5 mg/mL of ixekizumab; about 50±5 mM of acetate; about 12.5±1 mM of lysine; about 7.5±1 % (w/v) of trehalose; about 0.03±0.005 % (w/v) of polysorbate 80, wherein a pH is about 5.7±0.1;
17) a liquid formulation comprising about 80±5 mg/mL of ixekizumab; about 30±5 mM of acetate; about 17.5±1 mM of lysine; about 7.5±1 % (w/v) of trehalose; about 0.03±0.005 % (w/v) of polysorbate 80, wherein a pH is about 5.7±0.1; or
18) a liquid formulation comprising about 80±5 mg/mL of ixekizumab; about 30±5 mM of acetate; about 12.5±1 mM of lysine; about 10.5±1 % (w/v) of trehalose; about 0.03±0.005 % (w/v) of polysorbate 80, wherein a pH is about 5.7±0.1.

### (9) Device

According to another aspect, a device comprises the liquid formulation according to the one aspect described above.

The device is mainly used for parenteral administration (e.g.: subcutaneous, intramuscular, intravenous, intraperitoneal, intracerebrospinal, intraarticular, intrasynovial, and/or intrathecal administration). The device may be accompanied by instructions for administration.

The device may comprise the liquid formulation in a container selected from a syringe, a pre-filled syringe, an autoinjector, a bottle, a vial, and a tube.

In one embodiment, the device may comprise the liquid formulation in a pre-filled syringe. The pre-filled syringe may be a single-dose pre-filled syringe.

In one embodiment, the device may comprise the liquid formulation in an autoinjector. The pre-filled syringe may be a single-dose autoinjector.

### (10) Treatment of Disease

According to another aspect, a method of treating an IL-17-related condition comprises administering the liquid formulation according to the one aspect to a subject in need thereof. The liquid formulation may be in a form contained in the device.

According to another aspect, a use of the liquid formulation according to the one aspect is provided in the manufacture of a medicament for treating an IL-17-related condition.

The method of treating an IL-17-related condition may further comprise identifying a subject in need of administration of the anti-IL-17 antibody (e.g.: ixekizumab) before administering anti-IL-17 antibody.

The subject may be a subject in need of administering the liquid formulation containing the anti-IL-17 antibody (e.g.: ixekizumab). The subject in need of administering the liquid formulation containing the anti-IL-17 antibody (e.g.: ixekizumab) may be a subject having a disease or disorder that may be significantly treated (e.g.: symptoms may be eliminated, reduced, alleviated, improved, and the like) by administering the anti-IL-17 antibody. The subject may be selected from mammals comprising humans.

The liquid formulation may be administered in a pharmaceutically effective amount.

The IL-17-related condition may comprise any condition or disease that may be treated by administering the anti-IL-17 antibody. The IL-17-related condition which may be treated by administration of an anti-IL-17 antibody (e.g., ixekizumab) may comprise all indications previously approved for the anti-IL-17 antibody (e.g., ixekizumab) or all indications which may be approved in the future.

The IL-17-related condition may be selected from rheumatoid arthritis, psoriasis, ankylosing spondylitis, psoriatic arthritis, multiple myeloma, pruritus, palmoplantar pustulosis, and non-radiographic axial spondyloarthritis medication (nr-axSpA).

The psoriasis may be plaque psoriasis. The plaque psoriasis may be moderate to severe plaque psoriasis.

The psoriasis may be genital psoriasis.

The psoriatic arthritis may be active psoriatic arthritis (PsA).

### (11) Administration Route and Dosage

A liquid formulation according to one aspect may be administered via a parenteral route. The parenteral route may comprise subcutaneous administration, intravenous administration, and the like. Parenteral administration may be performed by bolus injection or continuous infusion.

In certain embodiment, the liquid formulation may be for subcutaneous injection.

The liquid formulation may be formulated into a formulation suitable for the administration route. For example, the liquid formulation may be formulated as an injection, an injectable ready-to-use form, and the like, but is not limited thereto.

The liquid formulation may be formulated such that the total amount or a pharmaceutically effective amount of the anti-IL-17 antibody (e.g.: ixekizumab) is contained in a single formulation, or divided into two or more formulations (e.g.: 2, 3, 4, 5, 6, 7, 8, 9, or 10 formulations). The liquid formulation may be provided in a single-dose form of a device.

The liquid formulation may be administered into the body such that the total amount of the anti-IL-17 antibody (e.g.: ixekizumab) contained in a single formulation is administered at once (e.g.: within 1 minute, within 30 seconds, within 20 seconds, or within 10 seconds); or is slowly administered into the body for 5 minutes or more, 10 minutes or more, 30 minutes or more, 60 minutes or more, 90 minutes or more, 120 minutes or more, 150 minutes or more, 180 minutes or more, 210 minutes or more, or 240 minutes or more, but is not limited thereto.

The subject for administering the liquid formulation may be selected from mammals comprising primates (e.g.: humans), rodents (e.g.: mice, rats, guinea pigs, hamsters, and rabbits), cats, dogs, pigs, cows, horses, and the like.

The pharmaceutically effective amount of the liquid formulation or the anti-IL-17 antibody (e.g.: ixekizumab) contained therein may indicate a content or dosage capable of exhibiting an effect on a desired pharmacological effect, such as eliminating, reducing, alleviating, or improving symptoms. The pharmaceutically effective amount may be determined in various ways by factors such as the formulation method, administration mode, age, weight, gender, pathological condition (severity of the condition), food, administration time, administration interval, administration route, excretion rate, response sensitivity, previous therapy, and clinical history of a patient. The dosage may be adjusted according to the judgment of a physician. The pharmaceutically effective amount may be administered at once, or may be divided and administered over two or more multiple times.

For example, the liquid formulation may be administered once or twice per 2 to 4 weeks over a period of 4 weeks or longer at a dose such that a concentration of an anti-IL-17 antibody (e.g., ixekizumab) is about 300 mg or less, about 240 mg or less, about 200 mg or less, about 160 mg or less, about 100 mg or less, about 80 mg or less, about 50 mg or less, about 25 mg, about 10 mg, or about 5 mg.

The liquid formulation may be produced as a general bulk formulation, where the components of the liquid formulation are adjusted to a higher concentration than the concentration required for administration, and may be used by being appropriately diluted before administration.

### Advantageous Effects of Invention

A liquid formulation of an anti-IL-17 antibody according to an aspect may have improved solubility and stability compared to conventional commercially available formulations. Therefore, the liquid formulation may be effectively used as a medicament for treating IL-17-related conditions.

### Brief Description of Drawings

FIG. 1 is a graph showing results of measuring T_{agg} for 16 formulations in which various types of buffers and pH are combined and a Taltz^{®} formulation.
FIG. 2 is a graph showing results of measuring T_{agg} for 5 formulations having various types of stabilizers, 1 formulation free of stabilizer, and a Taltz^{®} formulation.

### Best Mode for Carrying out the Invention

### Mode for the Invention

Hereinafter, the present disclosure will be described in more detail with reference to the following examples. However, the following examples are merely presented by exemplify the present disclosure, and the scope of the present disclosure is not limited thereto.

### [Experimental Methods]

### 1. Method of Measuring Opalescence

For 17 formulations comprising an anti-IL-17 antibody in which various types of buffers and pHs are combined, 7 formulations comprising an anti-IL-17 antibody and having various types of stabilizers, or 1 formulation comprising an anti-IL-17 antibody and free of a stabilizer, opalescence was determined to had occurred in the case where a color of a formulation changed to white as visually observed within 1 week of storage at room temperature or under refrigeration after manufacture of the formulation.

### 2. Method of Measuring Phase Separation

For 17 formulations comprising an anti-IL-17 antibody in which various types of buffers and pHs are combined, 7 formulations comprising an anti-IL-17 antibody and having various types of stabilizers, or 1 formulation comprising an anti-IL-17 antibody and free of a stabilizer, a phase separation was determined to had occurred in the case where a high-density protein layer is visually found on a bottom in a container within 1 week of storage at room temperature or under refrigeration after manufacture of the formulation.

### 3. Determination of Aggregation Temperature (T_{agg})

For 17 formulations comprising an anti-IL-17 antibody in which various types of buffers and pHs are combined, 7 formulations comprising an anti-IL-17 antibody and having various types of stabilizers, or 1 formulation comprising an anti-IL-17 antibody and free of a stabilizer, a radius of protein particles in a formulation was measured while increasing temperature from 25 °C to 80 °C at a rate of 25 °C/min using a high-throughput dynamic light scattering (HT-DLS) instrument. In this regard, the HT-DLS determined a temperature at a point where a radius of particles rapidly increased, as an aggregation temperature.

### 4. Measurement of Viscosity

For formulations comprising an anti-IL-17 antibody, having various pH, and comprising types of stabilizers, a viscosity of a formulation was measured at 20°C using an Initium (high-throughput viscometer) instrument from RheoSense. Specifically, after measuring 11 segments for the same sample, a viscosity value of each sample was calculated by obtaining an average of values having a slope fit R² value of 0.9995 or more. Considering that the formulation is injected into subcutaneous tissue, a lower viscosity is more advantageous in terms of patient convenience.

### 5. Measurement of %High Molecular Weight (%HMW)

For formulations containing an anti-IL-17 antibody and having various pH and types of stabilizers, a %HMW produced by a protein aggregation phenomenon was measured for samples under storage conditions of 40 °C or 25 °C by size-exclusion high performance liquid chromatography (SE-HPLC) from Waters. A temperature of a sampler in an instrument was maintained at 2 to 6 °C. A lower value of the %HMW indicates more advantageous in terms of protein stability.

### [Examples]

### Example 1: Screening of pH and Buffer Suitable for Liquid Formulation of Anti-IL-17 Antibody

### 1.1: Preparation of Formulations Comprising Various Types of Buffers and pH

Aqueous liquid formulations having composition ratios shown in Table 1 below were prepared by using ixekizumab (CAS No. 1143503-69-8) as an anti-IL-17 antibody.

Table 1 below shows compositions of 16 formulations prepared by combining types of buffers and pH being used in licensed biopharmaceuticals.

The antibody was included in a concentration range of 2 to 10 mg/mL, and the concentration was set in a different manner according to a required concentration of each analytical method. Specifically, the concentration of the antibody of each formulation was 8 mg/mL for measurement of opalescence, 10 mg/mL for measurement of phase separation, and 5 mg/mL for measurement of aggregation temperature.

**[Table 1]**

| No. | Concentration of antibody (mg/ml) | Buffer | pH | Stabilizer |
|---|---|---|---|---|
| 1-1 | 2 to 10 | 20 mM acetate | 4.0 | N/A^{**} |
| 1-2 | | 20 mM acetate | 4.5 | |
| 1-3 | | 20 mM acetate | 5.0 | |
| 1-4 | | 20 mM acetate | 5.5 | |
| 1-5 | | 20 mM acetate | 6.0 | |
| 1-6 | | 20 mM histidine | 5.0 | |
| 1-7 | | 20 mM histidine | 5.5 | |
| 1-8 | | 20 mM histidine | 6.0 | |
| 1-9 | | 20 mM citrate | 4.5 | |
| 1-10 | | 20 mM citrate | 5.0 | |
| 1-11 | | 20 mM citrate | 5.5 | |
| 1-12 | | 20 mM citrate | 6.0 | |
| 1-13 | | 20 mM succinate | 4.5 | |
| 1-14 | | 20 mM succinate | 5.0 | |
| 1-15 | | 20 mM succinate | 5.5 | |
| 1-16 | | 20 mM succinate | 6.0 | |
| 1-17^{*} | | Buffer-free | 5.7 | 8 % sucrose |

| | | | | |
|---|---|---|---|---|
| ^{*} 1-17 is a Taltz^{®} w/o citrate formulation. ^{**} N/A: Not applicable | | | | |

### 1.2: Screening of pH and Buffer for Improving Solubility of Liquid Formulation of Anti-IL-17 Antibody

To select pH conditions and buffers for improving solubility of a liquid formulation containing an anti-IL-17 antibody, experiments were performed as below based on the formulations manufactured in Example 1.1.

Specifically, ixekizumab was used as an example of the anti-IL-17 antibody. In addition, in order to confirm improvement of solubility, occurrence of opalescence and phase separation occurred was identified. A phenomenon in which the liquid formulation became cloudy after manufacture was visually confirmed as the occurrence of the opalescence. After storage of the prepared liquid formulation under 5 °C conditions for 1 week, occurrence of phase separation caused by a difference in protein density in a solution was visually confirmed.

Results of analyzing solubility of liquid formulations prepared by combining various buffers (acetate, histidine, citrate, and succinate) and pH conditions are shown in Table 2 below.

**[Table 2]**

| No. | Buffer, pH | Solubility | |
|---|---|---|---|
| | | Opalescence | Phase Separation |
| 1-1 | acetate, 4.0 | N | N |
| 1-2 | acetate, 4.5 | N | N |
| 1-3 | acetate, 5.0 | N | N |
| 1-4 | acetate, 5.5 | N | N |
| 1-5 | acetate, 6.0 | N | N |
| 1-6 | histidine, 5.0 | N | N |
| 1-7 | histidine, 5.5 | N | N |
| 1-8 | histidine, 6.0 | N | N |
| 1-9 | citrate, 4.5 | N | N |
| 1-10 | citrate, 5.0 | N | N |
| 1-11 | citrate, 5.5 | N | N |
| 1-12 | citrate, 6.0 | N | N |
| 1-13 | succinate, 4.5 | N | N |
| 1-14 | succinate, 5.0 | N | N |
| 1-15 | succinate, 5.5 | N | N |
| 1-16 | succinate, 6.0 | N | N |

As shown in Table 2 above, a phase separation caused by an interaction between anti-IL-17 antibodies or an opalescence in which a formulation becomes cloudy did not appear under the conditions of a total of 16 formulations, and thus, it was confirmed that the formulations were stable in terms of solubility.

### 1.3: Screening of pH and Buffer for Improving Stability of Liquid Formulation of Anti-IL-17 Antibody

To select pH conditions and buffers for improving stability of a liquid formulation containing an anti-IL-17 antibody, experiments were performed based on the formulations manufactured in Example 1.1 as below.

Specifically, ixekizumab was used as an example of the anti-IL-17 antibody, and in order to confirm improvement of stability, thermal stability of the liquid formulations was confirmed by measuring T_{agg}, which is a temperature at which aggregation is generated.

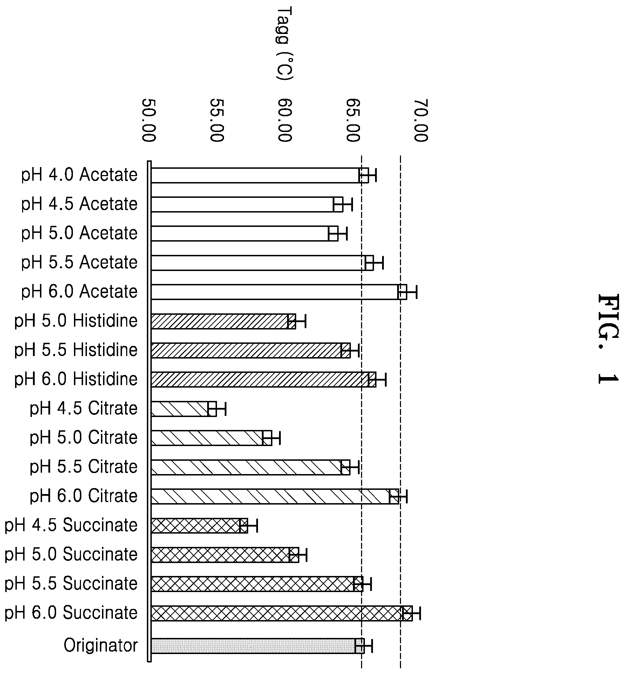

Results of analyzing thermal stability of liquid formulations prepared by combining various buffers (acetate, histidine, citrate, and succinate) and pH conditions are shown in Table 3 below and FIG. 1. In addition, as a control, a Taltz^{®} formulation (w/o citrate) known to comprise ixekizumab was used.

**[Table 3]**

| No. | Buffer, pH | T_{agg} (°C) |
|---|---|---|
| 1-1 | acetate, 4.0 | 66.22 [N=3, SD: 0.07] |
| 1-2 | acetate, 4.5 | 64.38 [N=3, SD: 0.14] |
| 1-3 | acetate, 5.0 | 63.98 [N=3, SD: 0.72] |
| 1-4 | acetate, 5.5 | 66.64 [N=3, SD: 0.22] |
| 1-5 | acetate, 6.0 | 69.12 [N=3, SD: 0.09] |
| 1-6 | histidine, 5.0 | 60.92 [N=3, SD: 0.53] |
| 1-7 | histidine, 5.5 | 64.91 [N=3, SD: 0.05] |
| 1-8 | histidine, 6.0 | 66.85 [N=3, SD: 0.14] |
| 1-9 | citrate, 4.5 | 54.98 [N=3, SD: 0.10] |
| 1-10 | citrate, 5.0 | 59.11 [N=3, SD: 0.37] |
| 1-11 | citrate, 5.5 | 64.88 [N=3, SD: 0.08] |
| 1-12 | citrate, 6.0 | 68.45 [N=3, SD: 0.19] |
| 1-13 | succinate, 4.5 | 57.35 [N=3, SD: 0.34] |
| 1-14 | succinate, 5.0 | 61.06 [N=3, SD: 0.42] |
| 1-15 | succinate, 5.5 | 65.82 [N=3, SD: 0.33] |
| 1-16 | succinate, 6.0 | 69.44 [N=3, SD: 0.25] |
| 1-17 | Bufferless, 5.7 (Taltz^{®} w/o Citrate - Originator 2) | 65.86 [N=3, SD: 0.64] |

As shown in Table 3 and FIG. 1, it was confirmed that among a total of 16 formulation conditions, 7 formulation conditions had a T_{agg} equal to or increased compared to the T_{agg} (65.86 °C) of a Taltz^{®} condition (pH 5.7, free of a buffer). Specifically, it was confirmed that the T_{agg} was increased in the case where acetate or succinate was used as a buffer and a pH condition was 5.5 and 6.0.

Therefore, as the formulation conditions in which the T_{agg} is increased, it was confirmed that a formulation using acetate or succinate as a buffer, particularly under a condition of pH 5.5 to 6.0, is a formulation having improved thermal stability compared to Taltz^{®}, which is a conventional anti-IL-17 antibody formulation.

### Example 2: Screening of Stabilizer Suitable for Liquid Formulation of Anti-IL-17 Antibody

### 2.1: Preparation of Formulations Comprising Various Types of Stabilizers

Aqueous liquid formulations having composition ratios shown in Table 4 below were prepared by using ixekizumab (CAS No. 1143503-69-8) as an anti-IL-17 antibody.

Table 4 below shows compositions of 5 formulations prepared by applying types of stabilizers being used in approved biopharmaceuticals and 1 formulation free of a stabilizer.

The antibody was included in a concentration range of 2 to 10 mg/mL, and the concentration was set in a different manner according to a required concentration of each analytical method. Specifically, the concentration of the antibody of each formulation was 8 mg/mL for measurement of opalescence, 10 mg/mL for measurement of phase separation, and 5 mg/mL for measurement of aggregation temperature.

**[Table 4]**

| No. | Concentration of antibody (mg/ml) | Buffer | pH | Stabilizer |
|---|---|---|---|---|
| 2-1 | 2 to 10 | acetate | 6.0 | 140 mM arginine |
| 2-2 | | | | 135 mM lysine |
| 2-3 | | | | 70 mM histidine |
| 2-4 | | | | 90 mM methionine |
| 2-5 | | | | 250 mM glycine |
| 2-6 | | | | N/A^{**} |
| 2-7^{*} | 2 to 10 | Buffer-free | 5.7 | 8 % sucrose |

| | | | | |
|---|---|---|---|---|
| ^{*} 2-7 is a Taltz^{®} w/o citrate formulation. ^{**} N/A: Not applicable | | | | |

### 2.2: Screening of Stabilizer for Improving Solubility of Liquid Formulation of Anti-IL-17 Antibody

To select stabilizers for improving solubility of a liquid formulation containing an anti-IL-17 antibody, experiments were performed based on the formulations manufactured in Example 2.1 as below.

Specifically, ixekizumab was used as an example of the anti-IL-17 antibody. In addition, in order to confirm improvement of solubility, the occurrence of opalescence and phase separation was evaluated. A phenomenon in which the liquid formulation became cloudy after manufacture was visually confirmed as the occurrence of the opalescence. After storage of the prepared liquid formulation under 5 °C conditions for 1 week, occurrence of a phase separation by a difference in protein density in a solution was visually confirmed.

Results of analyzing the solubility of liquid formulations prepared using various stabilizers (arginine, lysine, histidine, methionine, glycine, or stabilizer-free) are shown in Table 5 below.

Meanwhile, in the case of the liquid formulations of Table 5 below, all of pH and buffer conditions were manufactured identically to a condition of acetate (pH 6.0).

**[Table 5]**

| No. | Stabilizer | Solubility | |
|---|---|---|---|
| | | Opalescence | Phase Separation |
| 2-1 | 140 mM arginine | N | N |
| 2-2 | 135 mM lysine | N | N |
| 2-3 | 70 mM histidine | N | N |
| 2-4 | 90 mM methionine | N | N |
| 2-5 | 250 mM glycine | N | N |
| 2-6 | Stabilizer free | N | N |

As shown in Table 5 above, a phase separation or an opalescence in which a formulation becomes cloudy due to an interaction between anti-IL-17 antibodies did not appear under the conditions of 6 formulations, and thus, it was confirmed that the formulations were stable in terms of solubility.

### 2.3: Screening of Stabilizer for Improving Stability of Liquid Formulation of Anti-IL-17 Antibody

To select stabilizers for improving stability of a liquid formulation containing an anti-IL-17 antibody, experiments were performed based on the formulations manufactured in Example 2.1 as below.

Ixekizumab was used as an example of the anti-IL-17 antibody, and in order to confirm improvement of stability, thermal stability of the liquid formulations was confirmed by measuring T_{agg}, which is a temperature at which aggregation is generated.

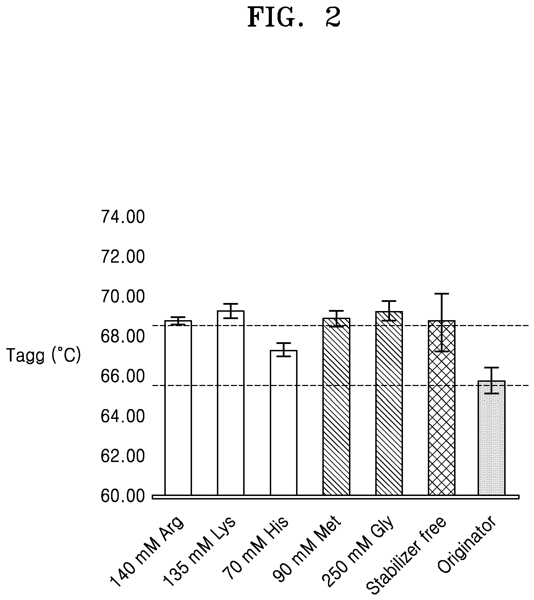

Results of analyzing thermal stabilizer of liquid formulations prepared using various stabilizers (arginine, lysine, histidine, methionine, glycine, or stabilizer-free) are shown in Table 6 below and FIG. 2. In addition, as a control, a Taltz^{®} formulation (w/o citrate) known to comprise ixekizumab was used.

Meanwhile, in the case of the liquid formulations except for Taltz^{®} of Table 6 below, all of pH and buffer conditions were manufactured identically to an acetate condition (pH 6.0).

**[Table 6]**

| No. | Stabilizer | T_{agg} (°C) |
|---|---|---|
| 2-1 | 140 mM arginine | 68.87 [N=3, SD: 0.19] |
| 2-2 | 135 mM lysine | 69.40 [N=3, SD: 0.37] |
| 2-3 | 70 mM histidine | 67.41 [N=3, SD: 0.35] |
| 2-4 | 90 mM methionine | 68.99 [N=3, SD: 0.39] |
| 2-5 | 250 mM glycine | 69.36 [N=3, SD: 0.50] |
| 2-6 | Stabilizer free | 68.81 [N=3, SD: 1.47] |
| 2-7 | 8 % sucrose (Taltz^{®} w/o Citrate - Originator 2) | 65.86 [N=3, SD: 0.64] |

As described in Table 6 and FIG. 2, it was confirmed that among the total of 6 formulation conditions, it was confirmed that the T_{agg} increased in all 6 formulation conditions, compared to the T_{agg} (65.86 °C) of the Taltz^{®} condition (pH 5.7, free of a buffer). Particularly, it was confirmed that the T_{agg} was excellent in the case where lysine was used as the stabilizer.

Therefore, it was confirmed that the formulation conditions in which the T_{agg} is increased are formulations having improved thermal stability compared to Taltz^{®}, which is a conventional anti-IL-17 antibody formulation.

### Example 3: Screening of Liquid Formulation of Anti-IL-17 Antibody with Improved Stability (1)

### 3.1: Evaluation of Viscosity of Candidate Formulations Comprising Combinations of Various pHs, Buffers, and Stabilizers

Based on the results of Examples 1 and 2, the following experiments were performed to derive a combination of a buffer and a stabilizer for improving the stability of a liquid formulation of an anti-IL-17 antibody.

Specifically, aqueous liquid formulations having composition ratios shown in Table 7 below were prepared by using ixekizumab (CAS No. 1143503-69-8) as an anti-IL-17 antibody, and viscosity of the formulations was measured.

Meanwhile, the antibody was included at a concentration of 80 mg/mL, and 0.03 % polysorbate 80 (PS 80) was used as the surfactant. Also, lysine hydrochloride (Lys-HCl) was used as the lysine.

**[Table 7]**

| No. | pH | Buffer | Stabilizer 1 | Stabilizer 2 | Viscosity (cP) |
|---|---|---|---|---|---|
| 3-1 | 6.0 | 20 mM sodium acetate | 49 mM lysine | 3 % mannitol | 4 |
| 3-2 | | | 49 mM lysine | 6 % trehalose | 5 |
| 3-3 | | | 4.70 % mannitol | - | 5 |
| 3-4 | | 20 mM sodium succinate | 49 mM lysine | 3 % mannitol | 4 |
| 3-5 | | | 49 mM lysine | 6 % trehalose | 4 |
| 3-6 | | | 4.70 % mannitol | - | 5 |
| 3-7^{*} | 5.7 | Buffer-free | 8 % sucrose | - | 6 |

| | | | | | |
|---|---|---|---|---|---|
| ^{*} 3-7 is a Taltz^{®} w/o citrate formulation. | | | | | |

As a result, as shown in Table 7, it was confirmed that all six formulation conditions exhibited reduced viscosity compared to the formulation under the Taltz^{®} condition (pH 5.7, free of a buffer). In particular, it was confirmed that the viscosity was improved and decreased when lysine was used as a stabilizer.

### 3.2: Evaluation of Stability of Candidate Formulations Comprising Combinations of Various pHs, Buffers, and Stabilizers

Aqueous liquid formulations having composition ratios shown in Table 8 below were prepared by using ixekizumab (CAS No. 1143503-69-8) as an anti-IL-17 antibody, and high-molecular weight % (%HMW) of the formulations was identified over time during storage under 40 °C or 25 °C conditions.

Meanwhile, the antibody was included at a concentration of 80 mg/mL, and 0.03 % polysorbate 80 (PS 80) was used as the surfactant. Also, lysine hydrochloride (Lys-HCl) was used as the lysine.

**[Table 8]**

| No. | pH | Buffer | Stabilizer 1 | Stabilizer 2 | %HMW (40 °C, 4wk) | %HMW (25 °C, 4wk) |
|---|---|---|---|---|---|---|
| 4-1 | 6.0 | 20 mM sodium acetate | 49 mM lysine | 3 % mannitol | 3.4 | 2.1 |
| 4-2 | | | 49 mM lysine | 6 % trehalose | 3.3 | 2.1 |
| 4-3 | | | 137 mM sodium chloride | - | 4.1 | 2.6 |
| 4-4 | | | 4.70 % mannitol | - | 4.2 | 2.5 |
| 4-5 | | 20 mM sodium succinate | 49 mM lysine | 3 % mannitol | 3.3 | 2.1 |
| 4-6 | | | 137 mM sodium chloride | - | 4.1 | 2.4 |
| 4-7 | | | 4.70 % mannitol | - | 4.1 | 2.5 |
| 4-8^{*} | 5.7 | Buffer-free | 8 % sucrose | - | 4.3 | 2.3 |

| | | | | | | |
|---|---|---|---|---|---|---|
| ^{*} 4-8 is a Taltz^{®} w/o citrate formulation. | | | | | | |

As a result, as shown in Table 8, it was confirmed that after 4 weeks under 40 °C conditions, all seven formulation conditions exhibited %HMW that was equivalent to or lower than that of the Taltz^{®} formulation (pH 5.7, free of a buffer). In addition, after 4 weeks under 25 °C conditions, 3 formulation conditions showed %HMW equivalent to or lower than that of the Taltz^{®} formulation (pH 5.7, free of a buffer).

In particular, in the case of the formulations using lysine as a stabilizer, it was confirmed that the %HMW was lower than that of the Taltz^{®} formulation (pH 5.7, free of a buffer) after 4 weeks at both 40 °C and 25 °C.

### Example 4: Screening of Liquid Formulation of Anti-IL-17 Antibody with Improved Stability (2)

Aqueous liquid formulations having composition ratios shown in Table 9 below were prepared by using ixekizumab (CAS No. 1143503-69-8) as an anti-IL-17 antibody.

Table 9 below shows compositions of 27 types of formulations prepared by applying combinations of various pH and concentration conditions, as combinations using sodium acetate, which is an example of acetate, as a buffer and one or more selected from the group consisting of lysine, trehalose, mannitol, and arginine as a stabilizer.

Meanwhile, the antibody was included at a concentration of 80 mg/mL, and 0.03 % polysorbate 80 (PS 80) was used as the surfactant. In addition, lysine hydrochloride (Lys-HCl) was used as the lysine, and arginine hydrochloride (Arg-HCl) was used as the arginine.

**[Table 9]**

| **No.** | **pH** | **Buffer** | **Stabilizer 1** | **Stabilizer 2** |
|---|---|---|---|---|
| 5-1 | 5.4 | 20 mM sodium acetate | 10 mM lysine | 6 % trehalose |
| 5-2 | 6.0 | 20 mM sodium acetate | 10 mM lysine | 6 % trehalose |
| 5-3 | 5.4 | 40 mM sodium acetate | 10 mM lysine | 6 % trehalose |
| 5-4 | 6.0 | 40 mM sodium acetate | 10 mM lysine | 6 % trehalose |
| 5-5 | 5.4 | 20 mM sodium acetate | 15 mM lysine | 6 % trehalose |
| 5-6 | 6.0 | 20 mM sodium acetate | 15 mM lysine | 6 % trehalose |
| 5-7 | 5.4 | 40 mM sodium acetate | 15 mM lysine | 6 % trehalose |
| 5-8 | 6.0 | 40 mM sodium acetate | 15 mM lysine | 6 % trehalose |
| 5-9 | 5.4 | 20 mM sodium acetate | 10 mM lysine | 9 % trehalose |
| 5-10 | 6.0 | 20 mM sodium acetate | 10 mM lysine | 9 % trehalose |
| 5-11 | 5.4 | 40 mM sodium acetate | 10 mM lysine | 9 % trehalose |
| 5-12 | 6.0 | 40 mM sodium acetate | 10 mM lysine | 9 % trehalose |
| 5-13 | 5.4 | 20 mM sodium acetate | 15 mM lysine | 9 % trehalose |
| 5-14 | 6.0 | 20 mM sodium acetate | 15 mM lysine | 9 % trehalose |
| 5-15 | 5.4 | 40 mM sodium acetate | 15 mM lysine | 9 % trehalose |
| 5-16 | 6.0 | 40 mM sodium acetate | 15 mM lysine | 9 % trehalose |
| 5-17 | 5.7 | 30 mM sodium acetate | 12.5 mM lysine | 7.5 % trehalose |
| 5-18 | 5.1 | 30 mM sodium acetate | 12.5 mM lysine | 7.5 % trehalose |
| 5-19 | 6.3 | 30 mM sodium acetate | 12.5 mM lysine | 7.5 % trehalose |
| 5-20 | 5.7 | 10 mM sodium acetate | 12.5 mM lysine | 7.5 % trehalose |
| 5-21 | 5.7 | 50 mM sodium acetate | 12.5 mM lysine | 7.5 % trehalose |
| 5-22 | 5.7 | 30 mM sodium acetate | 7.50 mM lysine | 7.5 % trehalose |
| 5-23 | 5.7 | 30 mM sodium acetate | 17.5 mM lysine | 7.5 % trehalose |
| 5-24 | 5.7 | 30 mM sodium acetate | 12.5 mM lysine | 4.5 % trehalose |
| 5-25 | 5.7 | 30 mM sodium acetate | 12.5 mM lysine | 10.5 % trehalose |
| 5-26 | 5.7 | 30 mM sodium acetate | 12.5 mM lysine | 4.0 % mannitol |
| 5-27 | 5.7 | 30 mM sodium acetate | 12.5 mM lysine | 125 mM arginine |
| 5-28* | 5.7 | Buffer-free | 8 % sucrose | - |

| | | | | |
|---|---|---|---|---|
| * 5-28 is a Taltz^{®} w/o citrate formulation. | | | | |

### 4.2: Evaluation of Viscosity of Liquid Formulation of Anti-IL-17 Antibody

Viscosity of the liquid formulation prepared in Example 4.1 was analyzed regarding improvement of stability of the liquid formulation comprising the anti-IL-17 antibody.

**[Table 10]**

| No. | Viscosity (cP) |
|---|---|
| 5-1 | 5.0 |
| 5-2 | 5.3 |
| 5-3 | 4.7 |
| 5-4 | 4.8 |
| 5-5 | 5.2 |
| 5-6 | 5.3 |
| 5-7 | 4.9 |
| 5-8 | 4.5 |
| 5-9 | 5.3 |
| 5-10 | 5.4 |
| 5-11 | 5.0 |
| 5-12 | 4.9 |
| 5-13 | 5.7 |
| 5-14 | 5.6 |
| 5-15 | 4.9 |
| 5-16 | 4.9 |
| 5-17 | 5.1 |
| 5-18 | 4.9 |
| 5-19 | 6.4 |
| 5-20 | 5.5 |
| 5-21 | 4.6 |
| 5-22 | 5.0 |
| 5-23 | 5.1 |
| 5-24 | 5.2 |
| 5-25 | 5.6 |
| 5-26 | 4.7 |
| 5-27 | 3.0 |
| 5-28 | 6.0 |

As a result, as shown in Table 10, it was confirmed that all twenty seven formulation conditions exhibited reduced viscosity compared to the formulation under the Taltz^{®} condition (pH 5.7, free of a buffer). It was confirmed that viscosity of all of the formulations was improved as compared to the Taltz^{®}.

### 4.3: Evaluation of Stability of Liquid Formulation of Anti-IL-17 Antibody

In order to select a combination of a buffer and a stabilizer for improving the stability of a liquid formulation comprising an anti-IL-17 antibody, the rate of change in %HMW (Δ%HMW) was identified after storage for 4 weeks under 40 °C or 25 °C conditions, based on the formulations prepared in Example 4.1.

**[Table 11]**

| No. | pH | Concentr ation of buffer | Stabilizer 1 | Stabilizer 2 | Δ%HMW (40 °C, 0-4wk) | Δ%HMW (25 °C, 0-4wk) |
|---|---|---|---|---|---|---|
| 5-1 | 5.4 | 20 mM | 10 mM lysine | 6 % trehalose | 3.0 | 0.8 |
| 5-2 | 6.0 | 20 mM | 10 mM lysine | 6 % trehalose | 2.9 | 1.0 |
| 5-3 | 5.4 | 40 mM | 10 mM lysine | 6 % trehalose | 3.0 | 0.9 |
| 5-4 | 6.0 | 40 mM | 10 mM lysine | 6 % trehalose | 2.8 | 1.0 |
| 5-5 | 5.4 | 20 mM | 15 mM lysine | 6 % trehalose | 3.0 | 0.8 |
| 5-6 | 6.0 | 20 mM | 15 mM lysine | 6 % trehalose | 2.8 | 1.0 |
| 5-7 | 5.4 | 40 mM | 15 mM lysine | 6 % trehalose | 3.0 | 0.8 |
| 5-8 | 6.0 | 40 mM | 15 mM lysine | 6 % trehalose | 2.7 | 0.9 |
| 5-9 | 5.4 | 20 mM | 10 mM lysine | 9 % trehalose | 2.8 | 0.8 |
| 5-10 | 6.0 | 20 mM | 10 mM lysine | 9 % trehalose | 2.8 | 0.9 |
| 5-11 | 5.4 | 40 mM | 10 mM lysine | 9 % trehalose | 2.9 | 0.8 |
| 5-12 | 6.0 | 40 mM | 10 mM lysine | 9 % trehalose | 2.7 | 0.9 |
| 5-13 | 5.4 | 20 mM | 15 mM lysine | 9 % trehalose | 2.8 | 0.8 |
| 5-14 | 6.0 | 20 mM | 15 mM lysine | 9 % trehalose | 2.7 | 0.9 |
| 5-15 | 5.4 | 40 mM | 15 mM lysine | 9 % trehalose | 2.8 | 0.8 |
| 5-16 | 6.0 | 40 mM | 15 mM lysine | 9 % trehalose | 2.5 | 0.9 |
| 5-17 | 5.7 | 30 mM | 12.5 mM lysine | 7.5 % trehalose | 2.8 | 0.9 |
| 5-18 | 5.1 | 30 mM | 12.5 mM lysine | 7.5 % trehalose | 3.1 | 0.7 |
| 5-19 | 6.3 | 30 mM | 12.5 mM lysine | 7.5 % trehalose | 2.8 | 1.0 |
| 5-20 | 5.7 | 10 mM | 12.5 mM lysine | 7.5 % trehalose | 2.8 | 0.9 |
| 5-21 | 5.7 | 50 mM | 12.5 mM lysine | 7.5 % trehalose | 2.7 | 0.9 |
| 5-22 | 5.7 | 30 mM | 7.50 mM lysine | 7.5 % trehalose | 2.9 | 0.9 |
| 5-23 | 5.7 | 30 mM | 17.5 mM lysine | 7.5 % trehalose | 2.7 | 0.9 |
| 5-24 | 5.7 | 30 mM | 12.5 mM lysine | 4.5 % trehalose | 3.1 | 1.0 |
| 5-25 | 5.7 | 30 mM | 12.5 mM lysine | 10.5 % trehalose | 2.6 | 0.6 |
| 5-26 | 5.7 | 30 mM | 12.5 mM lysine | 4.0 % mannitol | 2.9 | 0.9 |
| 5-27 | 5.7 | 30 mM | 12.5 mM lysine | 125 mM arginine | 2.0 | 0.5 |
| 5-28 | Taltz^{®} w/o citrate formulation | | | | 3.2 | 1.2 |

First, the Δ%HMW levels were analyzed for 16 formulations to which respective combinations of pH (5.4 or 6.0), concentration of sodium acetate as a buffer (20 mM or 40 mM), concentration of Lys-HCl as a stabilizer (10 mM or 15 mM), and concentration of trehalose as a stabilizer (6.0 % or 9.0 %) were applied.

As a result, as shown in Table 12 below, it was confirmed that after 4 weeks under 40 °C conditions, all 16 formulation conditions exhibited a reduced Δ%HMW compared to the Taltz^{®} formulation (pH 5.7, free of a buffer), and after 4 weeks under 25 °C conditions, all 16 formulation conditions showed a reduced Δ%HMW compared to the Taltz^{®} formulation (pH 5.7, free of a buffer).

Therefore, it may be confirmed that when acetate is used as a buffer and lysine and trehalose are used as stabilizers, stability is superior to that of the conventional Taltz^{®} formulation.

**[Table 12]**

| No. | pH | Buffer Concentra tion | Lysine Concentr ation | Trehalose Concentrati on | Δ%HMW (40 °C, 0-4wk) | Δ%HMW (25 °C, 0-4wk) |
|---|---|---|---|---|---|---|
| 5-1 | 5.4 | 20 mM | 10 mM | 6.0 % | 3.0 | 0.8 |
| 5-2 | 6.0 | 20 mM | 10 mM | 6.0 % | 2.9 | 1.0 |
| 5-3 | 5.4 | 40 mM | 10 mM | 6.0 % | 3.0 | 0.9 |
| 5-4 | 6.0 | 40 mM | 10 mM | 6.0 % | 2.8 | 1.0 |
| 5-5 | 5.4 | 20 mM | 15 mM | 6.0 % | 3.0 | 0.8 |
| 5-6 | 6.0 | 20 mM | 15 mM | 6.0 % | 2.8 | 1.0 |
| 5-7 | 5.4 | 40 mM | 15 mM | 6.0 % | 3.0 | 0.8 |
| 5-8 | 6.0 | 40 mM | 15 mM | 6.0 % | 2.7 | 0.9 |
| 5-9 | 5.4 | 20 mM | 10 mM | 9.0 % | 2.8 | 0.8 |
| 5-10 | 6.0 | 20 mM | 10 mM | 9.0 % | 2.8 | 0.9 |
| 5-11 | 5.4 | 40 mM | 10 mM | 9.0 % | 2.9 | 0.8 |
| 5-12 | 6.0 | 40 mM | 10 mM | 9.0 % | 2.7 | 0.9 |
| 5-13 | 5.4 | 20 mM | 15 mM | 9.0 % | 2.8 | 0.8 |
| 5-14 | 6.0 | 20 mM | 15 mM | 9.0 % | 2.7 | 0.9 |
| 5-15 | 5.4 | 40 mM | 15 mM | 9.0 % | 2.8 | 0.8 |
| 5-16 | 6.0 | 40 mM | 15 mM | 9.0 % | 2.5 | 0.9 |
| 5-28 | Taltz^{®} w/o citrate formulation | | | | 3.2 | 1.2 |

Next, Δ%HMW levels were analyzed for 3 formulations in which the concentration of sodium acetate (30 mM), and the types and concentrations of Lys-HCl (12.5 mM) and trehalose (7.5 %) as stabilizers were fixed, and only the pH was varied (5.1, 5.7, or 6.3).

As a result, as shown in Table 13 below, it was confirmed that after 4 weeks under 40 °C conditions, all 3 formulation conditions exhibited a reduced Δ%HMW compared to the Taltz^{®} formulation (pH 5.7, free of a buffer), and after 4 weeks under 25 °C conditions, all 3 formulation conditions showed a reduced Δ%HMW compared to the Taltz^{®} formulation (pH 5.7, free of a buffer).

In addition, it was confirmed that the formulation having a pH condition of about 5.7 exhibited superior stability compared to the formulations under other pH conditions.

Based on the above results, it may be confirmed that when the pH is in the range of about 5.0 to 6.5, the stability is superior to that of the conventional Taltz^{®} formulation, and in particular, the stability is even more superior in the range of about 5.5 to 6.0.

**[Table 13]**

| No. | pH | Buffer Concentr ation | Lysine Concentratio n | Trehalose Concentrati on | Δ%HMW (40 °C, 0-4wk) | Δ%HMW (25 °C, 0-4wk) |
|---|---|---|---|---|---|---|
| 5-17 | 5.7 | 30 mM | 12.5 mM | 7.5 % | 2.8 | 0.9 |
| 5-18 | 5.1 | 30 mM | 12.5 mM | 7.5 % | 3.1 | 0.7 |
| 5-19 | 6.3 | 30 mM | 12.5 mM | 7.5 % | 2.8 | 1.0 |
| 5-28 | Taltz^{®} w/o citrate formulation | | | | 3.2 | 1.2 |

Next, Δ%HMW levels were analyzed for 3 formulations in which the pH (5.7), the types and concentrations of Lys-HCl (12.5 mM) and trehalose (7.5 %) as stabilizers were fixed, and only the concentrations of the sodium acetate was varied (30 mM, 10 mM, or 50 mM).

As a result, as shown in Table 14 below, it was confirmed that after 4 weeks under 40 °C conditions, all 3 formulation conditions exhibited a reduced Δ%HMW compared to the Taltz^{®} formulation (pH 5.7, free of a buffer), and after 4 weeks under 25 °C conditions, all 3 formulation conditions showed a reduced Δ%HMW compared to the Taltz^{®} formulation (pH 5.7, free of a buffer).

Based on the above results, it may be confirmed that in the case of the formulations in which the concentration of the buffer is in the range of about 10 mM to 50 mM, the stability is superior to that of the conventional Taltz^{®} formulation.

**[Table 14]**

| No. | pH | Buffer Concentra tion | Lysine Concentr ation | Trehalose Concentrati on | Δ%HMW (40 °C, 0-4wk) | Δ%HMW (25 °C, 0-4wk) |
|---|---|---|---|---|---|---|
| 5-17 | 5.7 | 30 mM | 12.5 mM | 7.5 % | 2.8 | 0.9 |
| 5-20 | 5.7 | 10 mM | 12.5 mM | 7.5 % | 2.8 | 0.9 |
| 5-21 | 5.7 | 50 mM | 12.5 mM | 7.5 % | 2.7 | 0.9 |
| 5-28 | Taltz^{®} w/o citrate formulation | | | | 3.2 | 1.2 |

Next, Δ%HMW levels were analyzed for 3 formulations in which the pH (5.7), the concentration of sodium acetate and the concentration of trehalose (7.5 %) as a stabilizer were fixed, and only the concentrations of Lys-HCl were varied (12.5 mM, 7.5 mM, and 17.5 mM).

As a result, as shown in Table 15 below, it was confirmed that after 4 weeks under 40 °C conditions, all 3 formulation conditions exhibited an equal or reduced Δ%HMW compared to the Taltz^{®} formulation (pH 5.7, free of a buffer), and after 4 weeks under 25 °C conditions, all 3 formulation conditions showed an equal or reduced Δ%HMW compared to the Taltz^{®} formulation (pH 5.7, free of a buffer).

Based on the above results, it may be confirmed that in the case of the formulations in which the concentration of Lys-HCl as a stabilizer is about 7.5 mM to 17.5 mM, the stability is superior to that of the conventional Taltz^{®} formulation.

**[Table 15]**

| No. | pH | Buffer Concentra tion | Lysine Concentr ation | Trehalose Concentrati on | Δ%HMW (40 °C, 0-4wk) | Δ%HMW (25 °C, 0-4wk) |
|---|---|---|---|---|---|---|
| 5-17 | 5.7 | 30 mM | 12.5 mM | 7.5 % | 2.8 | 0.9 |
| 5-22 | 5.7 | 30 mM | 7.5 mM | 7.5 % | 2.9 | 0.9 |
| 5-23 | 5.7 | 30 mM | 17.5 mM | 7.5 % | 2.7 | 0.9 |
| 5-28 | Taltz^{®} w/o citrate formulation | | | | 3.2 | 1.2 |

Next, Δ%HMW levels were analyzed for 3 formulations in which the pH (5.7), the concentration of sodium acetate (30 mM), and the concentration of Lys-HCl (12.5 %) as a stabilizer were fixed, and only the concentrations of trehalose were varied (7.5 %, 4.5 %, and 10.5 %).

As a result, as shown in Table 16 below, it was confirmed that after 4 weeks under 40 °C conditions, all 3 formulation conditions exhibited a reduced Δ%HMW compared to the Taltz^{®} formulation (pH 5.7, free of a buffer), and after 4 weeks under 25 °C conditions, all 3 formulation conditions showed an equal or reduced Δ%HMW compared to the Taltz^{®} formulation (pH 5.7, free of a buffer).

In addition, it was confirmed that the formulations having a trehalose concentration of 7.5 % or 10.5 % exhibited superior stability compared to the formulation having a trehalose concentration of 4.5 %.

Based on the above results, it may be confirmed that when the concentration of trehalose as a stabilizer is in the range of about 4.5 % to 10.5 %, the stability is superior to that of the conventional Taltz^{®} formulation, and in particular, the stability is even more superior when the concentration of trehalose is in the range of about 7.5 % to 10.5 %.

**[Table 16]**

| No. | pH | Buffer Concentra tion | Lysine Concentr ation | Trehalose Concentrati on | Δ%HMW (40 °C, 0-4wk) | Δ%HMW (25 °C, 0-4wk) |
|---|---|---|---|---|---|---|
| 5-17 | 5.7 | 30 mM | 12.5 mM | 7.5 % | 2.8 | 0.9 |
| 5-24 | 5.7 | 30 mM | 12.5 mM | 4.5 % | 3.1 | 1.0 |
| 5-25 | 5.7 | 30 mM | 12.5 mM | 10.5 % | 2.6 | 0.6 |
| 5-28 | Taltz^{®} w/o citrate formulation | | | | 3.2 | 1.2 |

Next, Δ%HMW levels were analyzed for 3 formulations in which the pH (5.7), the concentration of sodium acetate (30 mM), and the concentration of Lys-HCl as a stabilizer (12.5 mM) were fixed, while the type of additional stabilizer was varied (7.5 % trehalose, 4.0 % mannitol, and 125 mM Arg-HCl).

As a result, as shown in Table 17 below, it was confirmed that after 4 weeks under 40 °C conditions, all 3 formulation conditions exhibited an equal or reduced Δ%HMW compared to the Taltz^{®} formulation (pH 5.7, free of a buffer), and after 4 weeks under 25 °C conditions, all 3 formulation conditions showed an equal or reduced Δ%HMW compared to the Taltz^{®} formulation (pH 5.7, free of a buffer).

In addition, it was confirmed that the formulation in which the additionally included stabilizer was arginine exhibited superior stability compared to the formulations comprising trehalose or mannitol.

Based on the above results, it may be confirmed that formulations comprising lysine as a stabilizer and additionally comprising trehalose, mannitol, or lysine as an additional stabilizer provide superior stability to the conventional Taltz^{®} formulation, and in particular, the stability is even more superior when the additionally included stabilizer is lysine.

**[Table 17]**

| No. | pH | Buffer Concentra tion | Stabilizer 1 | Stabilizer 2 | Δ%HMW (40 °C, 0-4wk) | Δ%HM W (25 °C, 0-4wk) |
|---|---|---|---|---|---|---|
| 5-17 | 5.7 | 30 mM | 12.5 mM lysine | 7.5 % trehalose | 2.8 | 0.9 |
| 5-26 | 5.7 | 30 mM | 12.5 mM lysine | 4.0 % mannitol | 2.9 | 0.9 |
| 5-27 | 5.7 | 30 mM | 12.5 mM lysine | 125 mM arginine | 2.0 | 0.5 |
| 5-28 | Taltz^{®} w/o citrate formulation | | | | 3.2 | 1.2 |

The above description of the present disclosure is provided for the purpose of illustration, and it would be understood by those skilled in the art that various changes and modifications may be made without changing technical conception and essential features of the present disclosure. Thus, it is clear that the above-described examples of the present disclosure are illustrative in all aspects and do not limit the present disclosure.

## Claims

1. A liquid formulation, comprising:
an anti-IL-17 antibody; and
a buffer,
wherein the liquid formulation has a pH of about 4.0 to about 7.0.

2. The liquid formulation of claim 1, wherein the anti-IL-17 antibody is ixekizumab.

3. The liquid formulation of claim 1, wherein a concentration of the anti-IL-17 antibody is about 5 mg/ml to about 300 mg/ml.

4. The liquid formulation of claim 1, wherein a concentration of the anti-IL-17 antibody is about 25 mg/ml to about 200 mg/ml.

5. The liquid formulation of claim 1, wherein a concentration of the anti-IL-17 antibody is about 50 mg/ml to about 150 mg/ml.

6. The liquid formulation of claim 1, wherein a pH of the liquid formulation is about 5.0 to about 6.5.

7. The liquid formulation of claim 1, wherein a pH of the liquid formulation is about 5.5 to about 6.0.

8. The liquid formulation of claim 1, wherein the buffer comprises one or more selected from histidine, phosphate, malate, tartrate, succinate, citrate, acetate, carbonate, salts thereof, and hydrates thereof.

9. The liquid formulation of claim 1, wherein a concentration of the buffer is about 1 mM to about 60 mM.

10. The liquid formulation of claim 1, wherein a concentration of the buffer is about 10 mM to about 50 mM.

11. The liquid formulation of claim 1, wherein a concentration of the buffer is about 30 mM.

12. The liquid formulation of claim 1, wherein the buffer comprises one or more selected from acetate, histidine, citrate, succinate, salts thereof, and hydrates thereof.

13. The liquid formulation of claim 1, wherein the buffer comprises one or more selected from acetate, histidine, succinate, salts thereof, and hydrates thereof.

14. The liquid formulation of claim 1, wherein the buffer is free of one or more selected from citrate, salts thereof, and hydrates thereof.

15. The liquid formulation of claim 1, wherein the buffer
1) is free of one or more selected from citrate, salts thereof, and hydrates thereof, and
2) comprises one or more selected from acetate, histidine, succinate, salts thereof, and hydrates thereof.

16. The liquid formulation of claim 1, comprising, as a buffer, one selected from acetate, salts thereof, and hydrates thereof, and having a pH of about 4.0 to about 6.0.

17. The liquid formulation of claim 1, comprising, as a buffer, one selected from histidine, salts thereof, and hydrates thereof, and having a pH of about 5.0 to about 6.0.

18. The liquid formulation of claim 1, comprising, as a buffer, one selected from citrate, salts thereof, and hydrates thereof, and having a pH of about 4.5 to about 6.0.

19. The liquid formulation of claim 1, comprising, as a buffer, one selected from succinate, salts thereof, and hydrates thereof, and having a pH of about 4.5 to about 6.0.

20. The liquid formulation of any one of claims 1 to 19, further comprising a stabilizer.

21. The liquid formulation of claim 20, wherein the stabilizer comprises one or more selected from sugars, sugar alcohols, amino acids, and metal salts.

22. The liquid formulation of claim 21, wherein the sugars comprise one or more selected from trehalose, sucrose, galactose, mannose, maltose, lactose, fructose, and glucose.

23. The liquid formulation of claim 21, wherein the sugar alcohols comprise one or more selected from mannitol, sorbitol, xylitol, arabitol, erythritol, lactitol, maltitol, and inositol.

24. The liquid formulation of claim 21, wherein the amino acids comprise one or more selected from glycine, alanine, valine, leucine, isoleucine, proline, phenylalanine, tyrosine, tryptophan, serine, threonine, cysteine, methionine, asparagine, glutamine, lysine, arginine, histidine, aspartic acid, and glutamic acid.

25. The liquid formulation of claim 21, wherein the metal salts comprise one or more selected from NaCl, KCl, NaF, KBr, NaBr, Na₂SO₄, NaSCN, CaCl₂, MgCl₂, and K₂SO₄.

26. The liquid formulation of claim 21, wherein a concentration of the sugars is about 1.0 % (w/v) to about 20.0 % (w/v).

27. The liquid formulation of claim 21, wherein a concentration of the sugars is about 4.0 % (w/v) to about 12.0 % (w/v).

28. The liquid formulation of claim 21, wherein a concentration of the sugar alcohol is about 1.0 % (w/v) to about 20.0 % (w/v).

29. The liquid formulation of claim 21, wherein a concentration of the sugar alcohols is about 2.0 % (w/v) to about 6.0 % (w/v).

30. The liquid formulation of claim 21, wherein a concentration of the amino acids is about 1 mM to about 300 mM.

31. The liquid formulation of claim 21, wherein a concentration of the amino acids is about 5 mM to about 200 mM.

32. The liquid formulation of claim 21, wherein a concentration of the metal salts is about 1 mM to about 300 mM.

33. The liquid formulation of claim 21, wherein a concentration of the metal salts is about 50 mM to about 200 mM.

34. The liquid formulation of claim 20, wherein the stabilizer comprises one or more selected from trehalose, mannitol, arginine, lysine, histidine, methionine, glycine, and NaCl.

35. The liquid formulation of claim 20, comprising about 3.0 to about 12.0 % (w/v) of trehalose as a stabilizer.

36. The liquid formulation of claim 20, comprising about 1.0 to about 10.0 % (w/v) of mannitol as a stabilizer.

37. The liquid formulation of claim 20, comprising about 5 to about 20 mM of lysine as a stabilizer.

38. The liquid formulation of claim 20, comprising about 50 to about 200 mM of arginine as a stabilizer.

39. The liquid formulation of claim 20, comprising about 50 to about 90 mM of histidine as a stabilizer.

40. The liquid formulation of claim 20, comprising about 70 to about 110 mM of methionine as a stabilizer.

41. The liquid formulation of claim 20, comprising about 230 to about 270 mM of glycine as a stabilizer.

42. The liquid formulation of claim 20, comprising about 100 to about 200 mM of NaCl as a stabilizer.

43. The liquid formulation of any one of claims 1 to 42, further comprising a surfactant.

44. The liquid formulation of claim 43, wherein the surfactant comprises a nonionic surfactant.

45. The liquid formulation of claim 43, wherein the surfactant comprises one or more selected from polysorbate, poloxamer, and sorbitan esters of other fatty acids.

46. The liquid formulation of claim 43, wherein the surfactant comprises one or more selected from polysorbate 20, polysorbate 40, polysorbate 60, polysorbate 65, polysorbate 80, and polysorbate 85.

47. The liquid formulation of claim 43, wherein a concentration of the surfactant is about 0.001 % to about 0.2 %.

48. The liquid formulation of claim 1, wherein liquid-liquid phase separation (LLPS) does not occur in the liquid formulation.

49. The liquid formulation of claim 1, wherein one or more selected from opalescence and phase separation does not occur in the liquid formulation.

50. The liquid formulation of claim 1, wherein solubility is improved.

51. The liquid formulation of claim 1, wherein the liquid formulation has an aggregation temperature (T_{agg}) of about 66 °C or higher.

52. The liquid formulation of claim 1, wherein the liquid formulation has an aggregation temperature (T_{agg}) of about 68 °C or higher.

53. The liquid formulation of claim 1, wherein a %HMW of the antibody as measured after storage under 40 °C conditions for 4 weeks is less than 4.3 and/or a %HMW of the antibody as measured after storage under 25 °C conditions for 4 weeks is less than 2.3, and the liquid formulation is selected from the following:
A) the buffer comprises acetate, and a stabilizer comprises lysine and mannitol;
B) the buffer comprises acetate, and a stabilizer comprises lysine and trehalose; or
C) the buffer comprises succinate, and a stabilizer comprises lysine and mannitol.

54. The liquid formulation of claim 1, wherein a %HMW of the antibody as measured after storage under 40 °C conditions for 4 weeks is less than 4.3 and/or a %HMW of the antibody as measured after storage under 25 °C conditions for 4 weeks is less than 2.3, and the liquid formulation is selected from the following:
A) the buffer comprises 10 to 30 mM of acetate, a stabilizer comprises 40 to 60 mM of lysine and 2 to 4 % of mannitol, and the pH is about 5.5 to 6.5;
B) the buffer comprises 10 to 30 mM of acetate, a stabilizer comprises 40 to 60 mM of lysine and 4 to 8 % of trehalose, and the pH is about 5.5 to 6.5; or
C) the buffer comprises 10 to 30 mM of succinate, a stabilizer comprises 40 to 60 mM of lysine and 2 to 4 % of mannitol, and the pH is about 5.5 to 6.5.

55. The liquid formulation of claim 1, wherein a Δ%HMW of the antibody as measured after storage under 40 °C conditions for 4 weeks is less than 3.2 and/or a Δ%HMW of the antibody as measured after storage under 25 °C conditions for 4 weeks is less than 1.2, and the liquid formulation is selected from the following:
A) the buffer comprises acetate, and a stabilizer comprises lysine and trehalose;
B) the buffer comprises acetate, and a stabilizer comprises lysine and mannitol; or
C) the buffer comprises acetate, and a stabilizer comprises lysine and arginine.

56. The liquid formulation of claim 1, wherein a Δ%HMW of the antibody as measured after storage under 40 °C conditions for 4 weeks is less than 3.2 and/or a Δ%HMW of the antibody as measured after storage under 25 °C conditions for 4 weeks is less than 1.2, and the liquid formulation is selected from the following:
A) the buffer comprises 10 to 50 mM of acetate, a stabilizer comprises 5 to 20 mM of lysine and 3 to 12 % (w/v) of trehalose, and the pH is about 5.0 to 6.5;
B) the buffer comprises 10 to 50 mM of acetate, a stabilizer comprises 5 to 20 mM of lysine and 3.0 to 5.0 % (w/v) of mannitol, and the pH is about 5.0 to 6.5; or
C) the buffer comprises 10 to 50 mM of acetate, a stabilizer comprises 5 to 20 mM of lysine and 100 to 150 mM of arginine, and the pH is about 5.0 to 6.5.

57. The liquid formulation of claim 1, wherein a viscosity of the liquid formulation is 2.0 cP to less than 6.0 cP.

58. The liquid formulation of claim 1, comprising:
about 5 mg/mL to about 300 mg/mL of ixekizumab;
about 10 mM to about 50 mM of acetate;
about 7.5 mM to about 12.5 mM of lysine;
about 100 mM to about 150 mM of arginine; and
about 0.01 %(w/v) to about 0.05 %(w/v) of polysorbate 80,
wherein the pH of the liquid formulation is about 5.4 to about 6.0.

59. The liquid formulation of claim 1, comprising:
about 60 mg/mL to about 100 mg/mL of ixekizumab;
about 20 mM to about 40 mM of acetate;
about 10 mM to about 15 mM of lysine;
about 110 mM to about 140 mM of arginine; and
about 0.02 % (w/v) to about 0.04 %(w/v) of polysorbate 80,
wherein the pH of the liquid formulation is about 5.6 to about 5.8.

60. The liquid formulation of claim 1, comprising:
about 5 mg/mL to about 300 mg/mL of ixekizumab;
about 10 mM to about 50 mM of acetate;
about 5 mM to about 20 mM of lysine;
about 3.0 % (w/v) to about 15.0 % (w/v) of trehalose; and
about 0.01 %(w/v) to about 0.05 %(w/v) of polysorbate 80,
wherein the pH of the liquid formulation is about 5.4 to about 6.0.

61. The liquid formulation of claim 1, comprising:
about 60 mg/mL to about 100 mg/mL of ixekizumab;
about 20 mM to about 40 mM of acetate;
about 10 mM to about 15 mM of lysine;
about 5.0 % (w/v) to about 10.0 % (w/v) of trehalose; and
about 0.02 % (w/v) to about 0.04 %(w/v) of polysorbate 80,
wherein the pH of the liquid formulation is about 5.6 to about 5.8.

62. The liquid formulation of any one of claims 1 to 61, wherein the liquid formulation is for subcutaneous injection.

63. A device comprising the liquid formulation of any one of claims 1 to 61.

64. The device of claim 63, wherein the liquid formulation is contained in a container selected from a syringe, a pre-filled syringe, an auto-injector, a bottle, a vial, and a tube.

65. The device of claim 63, wherein the liquid formulation is contained in a pre-filled syringe.

66. The device of claim 65, wherein the pre-filled syringe is a single-dose pre-filled syringe.

67. A method of treating an IL-17-related condition, the method comprising administering the liquid formulation of any one of claims 1 to 61 to a subject in need thereof.

68. The method of claim 67, wherein the IL-17-related condition is selected from rheumatoid arthritis, psoriasis, ankylosing spondylitis, psoriatic arthritis, multiple myeloma, pruritus, palmoplantar pustulosis, and non-radiographic axial spondyloarthritis medication (nr-axSpA).

69. A use of the liquid formulation of any one of claims 1 to 61 in the manufacture of a medicament for treating an IL-17-related condition.
